# FASCICULE DE BREVET EUROPEEN

(11) **EP 2 524 031 B1**
(45) Date de publication et mention de la délivrance du brevet: **08.07.2015**
(21) Numéro de dépôt: 11704276.2
(22) Date de dépôt: 11.01.2011
(51) Int. Cl.: C12N 5/071, G01N 33/50, C12N 5/074

(54) **LIGNEES DE CELLULES SOUCHES HUMAINES ET MURINES : MODELES DE PRECURSEURS DE CELLULES ENDOTHELIALES**
MENSCHLICHER STAMMZELLLINIEN UND MAUS: MODELL VON ENDOTHELIALEN PROGENITORZELLEN
HUMAN AND MOUSE STEM CELL LINES: MODEL OF ENDOTHELIAL PROGENITOR CELLS

(30) Priorité: 12.01.2010 FR 1000111
(43) Date de publication de la demande: 21.11.2012
(73) Titulaire: Centre National de la Recherche Scientifique (CNRS), 75794 Paris Cedex 16 (FR); Ludwik Hirszfeld Institute of Immunology and Experimental Therapy Polish Academy of Sciences, 53-114 Wroclaw (PL)
(72) Inventeur: KIEDA, Claudine, 45000 Orléans (FR); GRILLON, Catherine, 45150 Darvoy (FR); LAMERANT-FAYEL, Nathalie, 45160 Olivet (FR); PAPROCKA, Maria, 50501 Wroclaw (PL); KRAWCZENKO, Agnieszka, 53030 Wroclaw (PL); GOSZYK-DUS, Danuta, 53649 Wroclaw (PL)
(74) Mandataire: Novagraaf Technologies
(86) Numéro de dépôt international: PCT/FR2011/050045
(87) Numéro de publication internationale: WO 2011/086319

(56) Documents cités:
- MADONNA ROSALINDA ET AL: "In vitro neovasculogenic potential of resident adipose tissue precursors.", AMERICAN JOURNAL OF PHYSIOLOGY. CELL PHYSIOLOGY NOV 2008 LNKD- PUBMED:18787077, vol. 295, no. 5, novembre 2008 (2008-11), pages C1271-C1280, XP002592954, ISSN: 0363-6143
- KIM S ET AL: "Endothelial stem cells and precursors for tissue engineering: Cell source, differentiation, selection, and application", TISSUE ENGINEERING - PART B: REVIEWS 20080301 US LNKD- DOI:10.1089/TEB.2007.0304, vol. 14, no. 1, 1 mars 2008 (2008-03-01), pages 133-147, XP002592955, ISSN: 1937-3368
- HIDESUKE YAMAMOTO ET AL: "Identification of two distinct populations of endothelial progenitor cells differing in size and antigen expression from human umbilical cord blood", ANNALS OF HEMATOLOGY, SPRINGER, BERLIN, DE, vol. 87, no. 2, 2 octobre 2007 (2007-10-02), pages 87-95, XP019587270, ISSN: 1432-0584
- SHARPE EMERSON E III ET AL: "The origin and in vivo significance of murine and human culture-expanded endothelial progenitor cells", AMERICAN JOURNAL OF PATHOLOGY, vol. 168, no. 5, mai 2006 (2006-05), pages 1710-1721, XP002592956, ISSN: 0002-9440
- QIU HUI-YING ET AL: "Postnatal neovascularization by endothelial progenitor cells immortalized with the simian virus 40T antigen gene", INTERNATIONAL JOURNAL OF ONCOLOGY, vol. 28, no. 4, avril 2006 (2006-04), pages 815-821, XP002592959, ISSN: 1019-6439
- TARNOK ATTILA ET AL: "Phenotypes of Stem Cells from Diverse Origin", CYTOMETRY, vol. 77A, no. 1, 14 décembre 2009 (2009-12-14), pages 6-10, XP002592957,
- CIPRIANI P ET AL: "Impairment of endothelial cell differentiation from bone marrow-derived mesenchymal stem cells - New insight into the pathogenesis of systemic sclerosis", ARTHRITIS & RHEUMATISM, vol. 56, no. 6, juin 2007 (2007-06), pages 1994-2004, XP002592958, ISSN: 0004-3591
- REINISCH A ET AL: "Humanized large-scale expanded endothelial colony-forming cells function in vitro and in vivo", BLOOD 2009 AMERICAN SOCIETY OF HEMATOLOGY USA LNKD- DOI:10.1182/BLOOD-2008-09-181362, vol. 113, no. 26, 2009, pages 6716-6725, XP002592960,
- SCHREITER JESSICA ET AL: "Hematopoietic and endothelial progenitor cells from mouse adult testis derived stem cell lines.", BLOOD, vol. 108, no. 11, Part 1, novembre 2006 (2006-11), page 480A, XP009136489, & 48TH ANNUAL MEETING OF THE AMERICAN-SOCIETY-OF-HEMATOLOGY; ORLANDO, FL, USA; DECEMBER 09 -12, 2006 ISSN: 0006-4971
- BLEIZIFFER OLIVER ET AL: "T17b murine embryonal endothelial progenitor cells can be induced towards both proliferation and differentiation in a fibrin matrix.", JOURNAL OF CELLULAR AND MOLECULAR MEDICINE MAY 2009 LNKD- PUBMED:19538255, vol. 13, no. 5, mai 2009 (2009-05), pages 926-935, XP002592961, ISSN: 1582-4934
- AKESON ANN L ET AL: "In vitro model for developmental progression from vasculogenesis to angiogenesis with a murine endothelial precursor cell line, MFLM-4", MICROVASCULAR RESEARCH, vol. 61, no. 1, janvier 2001 (2001-01), pages 75-86, XP009136509, ISSN: 0026-2862
- MIYATA TAKASHI ET AL: "Platelet-derived growth factor-BB (PDGF-BB) induces differentiation of bone marrow endothelial progenitor cell-derived cell line TR-BME2 into mural cells, and changes the phenotype", JOURNAL OF CELLULAR PHYSIOLOGY, vol. 204, no. 3, septembre 2005 (2005-09), pages 948-955, XP002592962, ISSN: 0021-9541

## Description

### Domaine technique

La présente invention se rapporte à des cellules isolées humaines précurseurs des cellules endothéliales autres que les cellules souches embryonnaires, à des cellules isolées murines précurseurs des cellules endothéliales représentant un modèle de différentiation dans les deux cas. La présente invention se rapporte également à des procédés les utilisant. En particulier, la présente invention se rapporte à des lignées cellulaires établies de cellules isolées humaines précurseurs de cellules endothéliales autres que les cellules souches embryonnaires et à des lignées cellulaires établies de cellules isolées murines précurseurs de cellules endothéliales.

La présente invention trouve des applications dans le domaine médical et/ou vétérinaire, en particulier dans le domaine thérapeutique, et/ou dans le domaine de l'étude des mécanismes cellulaires, par exemple, des mécanismes cellulaires de réparation, d'angiogenèse ou de ciblage.

Dans la description ci-dessous, les références entre crochets (**[ ]**) renvoient à la liste des références présentées à la fin du texte.

### Etat de la technique

Les cellules précurseurs de cellules endothéliales font l'objet depuis plusieurs années de nombreuses études et publications scientifiques. En effet, depuis l'identification des cellules souches, plusieurs études ont été réalisées afin d'identifier les marqueurs spécifiques des cellules souches, ainsi que les différents éléments susceptibles d'induire une différentiation et/ou une spécialisation de ces cellules. Les cellules souches peuvent, avant différentiation, être à l'origine de l'ensemble des cellules. Elles peuvent se différencier et devenir, par exemple des précurseurs de cellules spécialisées. Un des types de cellules souches les plus connues sont les cellules souches hématopoïétiques. Ces cellules ont été très étudiées du fait de leur implication potentielle dans des maladies comme la leucémie myéloïde aigue, la croissance tumorale telle que présentée dans les documents Arbab AS, Pandit SD, Anderson SA, et al. « Magnetic résonance imaging and confocal microscopy studies of magnetically labeled endothelial progenitor cells trafficking to sites of tumor angiogenesis. » Stem Cells. 2006;24:671-678 **[1] ;** de Jonge-Peeters SD, Kuipers F, de Vries EG, Vellenga E. « ABC transporter expression in hematopoietic stem cells and the role in AML drug resistance. » Crit Rev Oncol Hematol. 2007 Jun; 62(3):214-26. Epub 2007 Mar 23. **[2]** ; Slayton WB, Li XM, Butler J, Guthrie SM, Jorgensen ML, Wingard JR, Scott EW. « The role of the donor in the repair of the marrow vascular niche following hematopoietic stem cell transplant. » Stem Cells. 2007 Nov;25(11):2945-55 **[3]**. Un des principaux objectifs de ces études a été d'identifier les différents précurseurs cellulaires, par exemple des précurseurs de cellules endothéliales.

Différentes études ont mis en avant des cellules avec différents marqueurs pouvant induire la formation de cellules endothéliales.

Les cellules progénitrices endothéliales (EPCs) peuvent être trouvées dans plusieurs tissus ainsi que dans la circulation sanguine comme le montre le document Dome B, Timar J, Ladanyi A, et al. « Circulating endothelial cells, bone marrow-derived endothelial progenitor cells and proangiogenic hematopoietic cells in cancer: From biology to therapy. » Crit Rev Oncol Hematol. 2009;69:108-1241 **[4].** Ces cellules sont dotées de la capacité à trouver activement les sites où il y a eu des dommages endothéliaux, d'adhérer à la surface endommagée et de se différencier en cellules endothéliales de manière à reconstituer l'intégrité vasculaire, les documents suivants présentent ces différentes actions Yamahara K, Itoh H. « Potential use of endothelial progenitor cells for regeneration of the vasculature. » Ther Adv Cardiovasc Dis. 2009;3:17-27. **[5]** ; Suh W, Kim KL, Kim JM, et al. « Transplantation of endothelial progenitor cellsaccelerates dermal wound healing with increased recruitment of monocytes/macrophages and neovascularization. » Stem Cells. 2005;23:1571-1578. **[6]** ; Slayton WB, Li XM, Butler J, et al. « The role of the donor in the repair of the marrow vascular niche following hematopoietic stem cell transplant. » Stem Cells.2007;25:2945-2955 **[7]**. Cet aspect de ciblage est particulièrement important pour la réparation de tissus, par exemple du tissu myocardique endommagé après un infarctus mais également des processus pathologiques tels que la croissance tumorale ou la rétinopathie **[1]**.

Les cellules précurseurs de cellules endothéliales ont été mises en évidence il y a plus de dix ans, cependant leur identification, leur caractérisation et leur potentiel de différentiation réel sont encore controversés.

Un des problèmes principaux pour l'identification des cellules précurseurs de cellules endothéliales provient de l'absence de marqueurs spécifiques et du manque de critères stricts pour les définir, ainsi que leurs fonctions.

Il n'existe pas actuellement dans l'art, de lignées cellulaires établies pour les cellules précurseurs de cellules endothéliales. Ainsi, aucune lignée cellulaire de cellules précurseurs de cellules endothéliales n'est disponible, quelque soit l'espèce d'origine.

Des progéniteurs de cellules endothéliales ou angioblastes putatifs ont été pour la première fois isolés du sang périphérique humain par sélection sur billes magnétiques en se basant sur l'expression sur la surface cellulaire de l'antigène CD347. *In vitro,* ces cellules ont été capables de se différencier en cellules endothéliales. Dans les modèles animaux de l'ischémie, les cellules précurseurs de cellules endothéliales sont incorporées dans les sites d'angiogenèse active, tel que présenté dans le document Shi Q, Rafii S, Wu MH, et al. « Evidence for circulating bone marrow-derived endothelial cells. » Blood. 1998;92:362-367 **[8]**.

L'identification des cellules précurseurs de cellules endothéliales est très difficile car il s'agit d'une population rare à la fois dans la moelle osseuse et dans la circulation sanguine.

Afin de procéder à la séparation de ces cellules, une sélection a été faite, dans l'art, via l'expression à la surface de marqueurs putatifs des cellules précurseurs de cellules endothéliales. Par exemple, une combinaison des marqueurs CD133 et/ou CD34 et parfois des marqueurs spécifiques des cellules endothéliales tels que VEGFR-2 (CD309), VE-Cadhérine (CD144) et la molécule d'adhésion de cellule de mélanome (M-CAM « melanoma cell adhesion molecule », CD146) ont été utilisés pour séparer les cellules souches et les précurseurs cellulaires comme décrits dans Lin Y, Weisdorf DJ, Solovey A, et al. « Origins of circulating endothelial cells and endothelial outgrowth from blood. » J Clin Invest. 2000;105:71-77 **[9]** ; Peichev M, Naiyer AJ, Pereira D, et al. « Expression of VEGFR-2 and AC133 by circulating human CD34(+) cells identifies a population of functional endothelial precursors. » Blood. 2000;95:952-958. [10]. Les cellules précurseurs de cellules endothéliales étaient alors propagées dans diverses conditions et caractérisées par des méthodes *in vitro* et/ou *in vivo* qui menaient à des résultats contradictoires.

Après l'étape de séparation, plusieurs marqueurs (CD45, CD13, CD31, CD105, CD144, CD202b, Ulex Agglutinin-1 (UEA-1)) étaient habituellement évalués afin de caractériser les populations de cellules obtenues et de discriminer les EPCs putatifs des cellules souches hématopoïétiques (HSCs). Les lignées endothéliales et hématopoïétiques ayant toutes les deux pour origine un progéniteur commun : l'hémangioblaste, et plus les progéniteurs sont proches, plus leurs similarités sont importantes et donc plus la distinction entre les lignées sera difficile.

Récemment, l'antigène leucocytaire commun CD45 a été suggéré comme marqueur pour aider à distinguer les lignées hématopoïétiques et endothéliales, puisqu'il n'est pas exprimé dans les hémangioblastes embryonnaires et est acquis durant la différentiation uniquement par les cellules de la lignée hématopoïétique. Il a également été démontré que chez les adultes, uniquement les cellules CD34+ CD45- étaient capables de se différencier vers les cellules précurseurs de cellules endothéliales tandis que les cellules CD34+ CD45+ se différencient plutôt en cellules de type EC exprimant le marqueur monocytique CD14+ additionnel.

Par ailleurs, les cellules progénitrices qui ont les marqueurs suivants : CD34+AC133+ et VEGFR2+ ne sont pas des cellules précurseurs de cellules endothéliales mais des cellules précurseurs de cellules hématopoïétiques comme indiqué dans Case J, Mead LE, Bessler WK, Prater D, White HA, Saadatzadeh MR, Bhavsar JR, Yoder MC, Haneline LS, Ingram DA « Human CD34+AC133+VEGFR-2+ cells are not endothelial progenitor cells but distinct, primitive hematopoietic progenitors. » Exp Hematol. 2007 Oct;35(10):1479-80 **[11]**.

Il existe donc clairement des doutes concernant les marqueurs des cellules précurseurs de cellules endothéliales et un réel besoin d'identifier et d'isoler des cellules précurseurs des cellules endothéliales et qui soient utilisables en thérapie.

Par ailleurs, tout comme les cellules souches hématopoïétiques, les précurseurs de cellules endothéliales sont connus pour leurs contributions à la génération de vaisseaux chez les adultes et la modulation de la vascularisation postnatale. Ces cellules sont également connues pour leur rôle dans la croissance des tumeurs via la génération de nouveaux vaisseaux. En effet, des études ont montré, via l'utilisation de dispositifs particuliers, que des cellules précurseurs pouvaient être recrutées au niveau de tumeurs et/ou de lésions tissulaires afin de produire des vaisseaux permettant ainsi la croissance de ces tumeurs et/ou la participation à la régénération des tissus lésés comme décrit dans Arbab AS, Pandit SD, Anderson SA et al. **[1]** ; Asahara T, Murohara T, Sullivan A, Silver M, van der Zee R, Li T, Witzenbichler B, Schatteman G, Isner JM. « Isolation of putative progenitor endothelial cells for angiogenesis. » Science. 1997 Feb 14;275(5302):964-7 **[12]**. Il n'existe cependant pas dans l'état de la technique de « modèle » de lignée cellulaire précurseur de cellule endothéliale.

Il existe un réel besoin de trouver au moins un modèle/lignée cellulaire établie palliant les défauts, inconvénients et obstacles de l'art antérieur, en particulier des lignées cellulaires permettant d'étudier les mécanismes cellulaires, par exemple ceux impliqués dans la génération de vaisseaux, des procédés permettant d'identifier des molécules agissant sur l'adressage des cellules, la formation de nouveaux vaisseaux et permettant de ainsi maîtriser, le mode de recrutement et les molécules impliquées et/ou inhibant ce recrutement de cellules et/ou la formation des vaisseaux, permettant d'autre part de réduire les coûts et d'améliorer les traitements de pathologies impliquant les cellules précurseurs de cellules endothéliales.

Il existe également donc un réel besoin d'identifier, d'isoler et de disposer de cellules précurseurs de cellules endothéliales, qui sont notamment utilisables en thérapie, par exemple en thérapie cellulaire pour le traitement de lésions tissulaires en ciblant spécifiquement le tissu lésé et/ou d'autres pathologies liées par exemple à des cellules génétiquement déficientes permettant ainsi maîtriser, d'améliorer et de diminuer le coût des traitements.

### Exposé de l'invention

La présente invention permet précisément de résoudre les problèmes et inconvénients précités de l'état de la technique en fournissant des cellules isolées précurseurs de cellules endothéliales.

Les inventeurs sont les premiers à avoir réussi à isoler des cellules humaines précurseurs de cellules endothéliales autre que les cellules souches embryonnaires, et des cellules murines précurseurs de cellules endothéliales.

En outre, les inventeurs ont immortalisé lesdites cellules et ont obtenu des lignées cellulaires établies, c'est-à-dire des lignées cellulaires immortalisées, stables, non tumorigènes et dont les caractéristiques sont identiques d'une génération à une autre. Une définition d'une lignée cellulaire établie est donnée dans le livre Gene IV, de Benjamin Leuvine, page1133, 6ème édition, De Boeck Université.

La présente invention a donc pour objet une cellule isolée précurseur des cellules endothéliales choisie parmi les cellules isolées humaines précurseur de cellules endothéliales déposées à la Collection Nationale de Culture de Microorganismes (CNCM), sous les numéros CNCM n° I-4220, n° I-4221 ou les cellules isolées murines déposées à la Collection Nationale de Culture de Microorganismes (CNCM), sous les numéros CNCM n° I-4222 n° I-4223.

Les inventeurs ont mis au point des lignées après la réalisation de la culture et stabilisation du phénotype de différentes cellules qui répondent à cette définition de l'invention. Ces cellules ont été déposées à la Collection Nationale de Cultures de Microorganisme (CNCM), Institut Pasteur, 25 rue du Docteur Roux, 75724 Paris cedex 15, France. Il s'agit des cellules isolées humaines précurseurs de cellules endothéliales autres que les cellules souches embryonnaires déposées à la Collection Nationale de Culture de Microorganismes (CNCM), Institut Pasteur, 25 rue du Docteur Roux, 75724 Paris cedex 15, France le 18 août 2009 sous les numéros CNCM n° I-4220 (HucPEC 55.1) et n° I-4221 (HucPEC 55.2).

Il s'agit de cellules isolées murines précurseurs de cellules endothéliales. déposées à la Collection Nationale de Culture de Microorganismes (CNCM), Institut Pasteur, 25 rue du Docteur Roux, 75724 Paris cedex 15, France le 18 août 2009 sous les numéros CNCM n° I-4222 (MAgEC 10.5) et n° I-4223 (MAgEC 11.5).

Parmi les nombreux avantages que les inventeurs ont mis en évidences concernant la présente invention, les cellules précitées permettent d'identifier des molécules susceptibles d'induire une différentiation et/ou une spécialisation. En particulier, les inventeurs ont mis en évidence que certaines molécules étaient susceptibles de stimuler les cellules pour provoquer une angiogenèse.

Aussi, la présente invention a également pour objet un procédé de criblage de molécules susceptibles d'induire une différentiation et/ou une spécialisation d'une cellule isolée telle que décrite précédemment comprenant les étapes de:
- introduction de la molécule à cribler dans un milieu adapté à la culture de ladite cellule,
- introduction d'au moins une cellule isolée dans ledit milieu,
- culture de ladite cellule dans ledit milieu pendant un temps suffisant pour permettre sa différentiation et/ou sa spécialisation,
- prélèvement d'au moins une cellule issue de ladite culture,
- observation de la différentiation et/ou spécialisation de ladite cellule par observation phénotypique de la cellule et/ou par révélation de marqueurs de différentiation.

Selon la présente invention, l'étape d'introduction de la molécule à cribler peut être réalisée antérieurement, simultanément et/ou postérieurement à l'étape d'introduction d'au moins une cellule isolée.

Dans la présente invention par « différentiation », il est entendu un mécanisme par lequel la cellule va acquérir des caractéristiques particulières. Par exemple, la différentiation peut conduire à l'obtention de cellule de type vasculaire, des cellules épithéliales pigmentées ou non pigmentées, des cellules de type ostéocyte, musculaire, adipocyte etc.

Dans la présente invention par « spécialisation», il est entendu un procédé par lequel la cellule va être spécialisée, c'est-à-dire que la cellule sera une cellule différenciée, par exemple des cellules endothéliales de vaisseaux, des cellules endothéliales circulantes, des cellules endothéliales de moelle osseuse, de la rate, du thymus, d'organes lymphoïdes secondaires, d'organes périphériques par exemple des cellules endothéliale du foie, du poumon, du rein ou du cerveau.

Dans la présente par « molécule à cribler», il est entendu une molécule chimique, un peptide et/ou toute molécule connue de l'homme du métier. Par exemple, la molécule à cribler peut être une molécule disponible dans le commerce et/ou pour laquelle on désire connaître son effet sur les cellules de la présente invention. Il peut s'agir également de molécules connues pour induire la différentiation ou la spécialisation cellulaire, par exemple des facteurs de croissance par exemple les membres de la famille des facteurs de croissance dérivée des plaquettes (PDGF : « Platelet derived growth factor »), le PDGF1, PDGF2, le facteur de croissance vasculaire (VEGF : « Vascular Endothelial growth factor »), le facteur de perméabilité vasculaire (VPF : « vascular permeability factor »), les membres de la famille du facteur de croissance épidermique (EGF : « Epidermal Growth Factor »), le facteur de croissance transformant (TGFa « Transforming Growth Factor a »), les membre de la famille des facteurs de croissance fibroblastique (FGF : « Fibroblast Growth Factor »), les membres de la famille des facteurs de croissance de l'insuline (IGF : « Insulin Growth Factor »), des facteurs de différentiation, par exemple, les Interleukines IL3, IL6, 8, IP 10, le VEGF, l'angiogénine, des cytokines recombinantes, par exemple le facteur recombinant de cellule souche (rh SCF : « stem cell factor »), le facteur recombinant de stimulation des colonies macrophages-granulocytes (rh GM-CSF : « Granulocyte Macrophage Colony Stimulating Factor » Il peut s'agir également de gènes thérapeutiques ciblés, de toute molécule active connue dans l'état de la technique et/ou de tout médicament indiqué dans le dictionnaire Vidal 2009 (marque enregistrée).

Dans la présente par « milieu adapté à la culture », il est entendu tout milieu connu de l'homme du métier qui permet la culture de cellules. Il peut s'agir par exemple d'un milieu liquide ou solide. Il peut s'agir, par exemple, de milieux disponibles dans le commerce, par exemple, un milieu du type aMEM, EBM-2, commercialisé par la société Clonetics, Lonza, Saint Beauzire, France, les milieux Basals de la société PromoCell. L'homme du métier grâce à ces connaissances générales saura aisément adapter et/ou supplémenter les milieux, si nécessaire, pour la culture des cellules. Par exemple, un milieu EBM-2 commercialisé par la société Clonetics, Lonza, Saint Beauzire, France) peut être supplémenté avec 10% de Sérum Foetal Bovin commercialisé par la société Hyclon, Longan, Utah, USA et au moins un facteur de croissance décrit dans le document, tels que l'ECGS (Endothelial cell growth supplement) et/ou de différentiation décrit dans le document (VEGF).

Selon un mode particulier de réalisation du procédé de l'invention, lesdites molécules induisent une différentiation et/ou spécialisation conduisant à l'angiogenèse. Ces molécules peuvent être par exemple des molécules pro ou anti angiogenèse.

Dans la présente, l'étape de culture peut être réalisée à une température comprise entre 20 et 40°C, de préférence à 37°C.

Dans la présente la culture de cellules peut être réalisée dans tout contenant connu de l'homme du métier, par exemple, il peut s'agir de boîtes de Pétri, de plaques 6 puits, 24 puits, 96 puits, de tube à essai, d'incubateur, par exemple « rollers », cultures sur supports tels que billes de Cytodex, boites en plastique Falcon et autres marques.

Dans la présente l'étape de prélèvement peut être réalisée par toute méthode connue de l'homme du métier. Par exemple, le prélèvement peut être réalisé via une pipette, un dispositif de prélèvement, par exemple un tube de prélèvement, préleveur de liquide à valve, tube de prélèvement par aspiration.

Dans la présente l'observation phénotypique peut être réalisée par toute méthode connue de l'homme du métier. Par exemple, elle peut être réalisée par observation directe, par observation via un microscope, par exemple un microscope électronique, microscope électronique, microscope électronique à balayage, microscope inversé, à contraste de phase, microscope de fluorescence, confocal, vidéo microscope.

De manière avantageuse, l'observation phénotypique peut être réalisée par vidéo microscopie ce qui permet de rendre le procédé de l'invention quantitatif.

La caractérisation phénotypique peut être réalisée par étude de la fixation d'anticorps spécifiques éventuellement marqués et/ou révélés par un second anticorps dirigé contre la molécule d'immunoglobuline concernée et fluorescent. Le marquage peut être révélé par cytofluorimétrie en flux.

Dans la présente la révélation de marqueurs de différentiation peut être réalisé par tout moyens connus de l'homme du métier, par exemple aux moyens de colorants spécifiques, d'anticorps spécifiques desdits marqueurs de différentiation tel que présenté dans le document pour les marquages cytochimiques, par exemple des anticorps disponibles dans le commerce, par exemple des anticorps monoclonaux, des anticorps polyclonaux, par exemple des anticorps monoclonaux de souris commercialisés par la société Sigma, la société Becton Dickinson Biosciences , par révélation fluorométrique utilisant des anticorps marqués et/ou une réaction enzymatique.

Avantageusement selon la présente invention, l'étape d'identification du phénotype et/ou des marqueurs permet de connaître l'effet de la molécule criblée sur la cellule isolée cultivée.

La présente invention a également pour objet un kit de mise en oeuvre du procédé décrit précédemment comprenant :
- au moins une cellule isolée selon l'invention, et
- un moyen de détection de la différentiation et/ou spécialisation cellulaire.

Dans la présente invention, un moyen de détection peut être tout moyen connu de l'homme du métier permettant par exemple d'observer visuellement les cellules, par exemple un moyen de détection optique, par exemple un microscope tel que décrit précédemment, par exemple un moyen de détection optique permettant de détecter des marqueurs de différentiation par exemple un anticorps contre un marqueur de différentiation et/ou tout moyen connu de l'homme du métier permettant de détecter la différentiation et/ou la spécialisation cellulaire. Il peut s'agir par exemple des anticorps décrits précédemment.

La présente invention a également pour objet une culture cellulaire comprenant au moins une cellule isolée décrite précédemment.

Dans la présente, la culture cellulaire peut être, par exemple toute culture de cellule comprenant au moins une cellule de l'invention, par exemple une cellule endothéliale précurseur humaine ou murine selon l'invention.

Les inventeurs ont découvert que les cellules isolées selon l'invention étaient capables de cibler des sites pathologiques et également de régénérer de nouveaux tissus endothéliaux. En particulier, les inventeurs ont découvert que les cellules selon l'invention sont capables de cibler spécifiquement des tumeurs et/ou des tissus lésés. Ainsi les cellules de l'invention peuvent apporter aux niveaux de sites pathologiques des molécules thérapeutiques et/ou des gènes. Les cellules de l'invention peuvent également par exemple cibler des tissus lésés et/ou nécrosés afin de régénérer lesdits tissus.

Ainsi, la présente invention a pour objet une cellule isolée selon l'invention pour l'utilisation comme médicament et/ou une utilisation la fabrication d'un médicament.

De préférence, le médicament est destiné au traitement des cellules génétiquement déficientes, des lésions tissulaires, de dommages au niveau de tissus endothéliaux, des tissus nécrosés. Par exemple, le médicament peut être destiné au traitement d'infarctus, de lésions vasculaires, de sites de dommages dus au diabète.

Selon la présente invention, les cellules isolées de l'invention peuvent être également utilisées comme transporteurs de gènes et/ou d'au moins une molécule thérapeutique vers un site pathologique. L'expression du gène peut être conditionnée aux conditions environnementales du site pathologique.

Avantageusement, l'adressage de gènes et/ou d'au moins une molécule thérapeutique à un site pathologique permet en outre d'améliorer l'efficacité de la molécule via une action directe et ciblée permettant par exemple de diminuer la quantité nécessaire pour obtenir l'effet thérapeutique et de diminuer l'apparition d'effets secondaire et/ou de diminuer les effets secondaires liés aux molécules

Dans la présente par « site pathologique », il est entendu par exemple toute anomalie tissulaire et/ou cellulaire, par exemple il peut s'agir d'une lésion tissulaire, des tissus nécrosés, cellules génétiquement déficientes, de tumeurs et/ou tout site de prolifération d'une pathologie. De préférence, il s'agit d'une lésion tissulaire, des tissus nécrosés, cellules génétiquement déficientes, de tumeurs, de blessures, d'une ischémie.

Dans la présente par « molécule thérapeutique », il est entendu toute molécule connue de l'homme du métier susceptible d'être utilisée pour le traitement d'une pathologie dans le domaine médical et/ou vétérinaire, il peut s'agir par exemple de toute molécule indiquée dans le dictionnaire Vidal 2009.

La présente invention trouve donc avantageusement des applications dans le domaine thérapeutique, par exemple vétérinaire et/ou médicale pour, par exemple le traitement de lésions tissulaire, l'administration de molécules thérapeutiques, mais également dans le domaine pharmaceutique dans l'identification de molécules susceptibles d'induire une différentiation et/ou une spécialisation de cellules précurseurs de cellules endothéliales...

D'autres avantages pourront encore apparaître à l'homme du métier à la lecture des exemples ci-dessous, illustrés par les figures annexées, donnés à titre illustratif.

### Brève description des figures

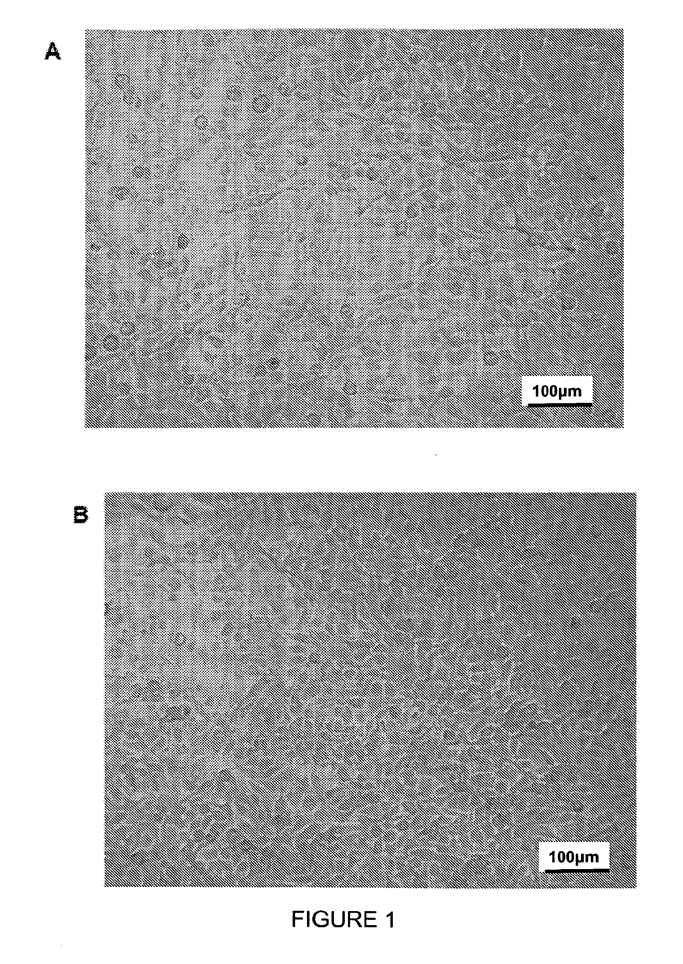

- La figure 1 est une photographie présentant la morphologie de deux lignées cellulaires HucPEC 55.1 (A) et HucPEC 55.2 (B)

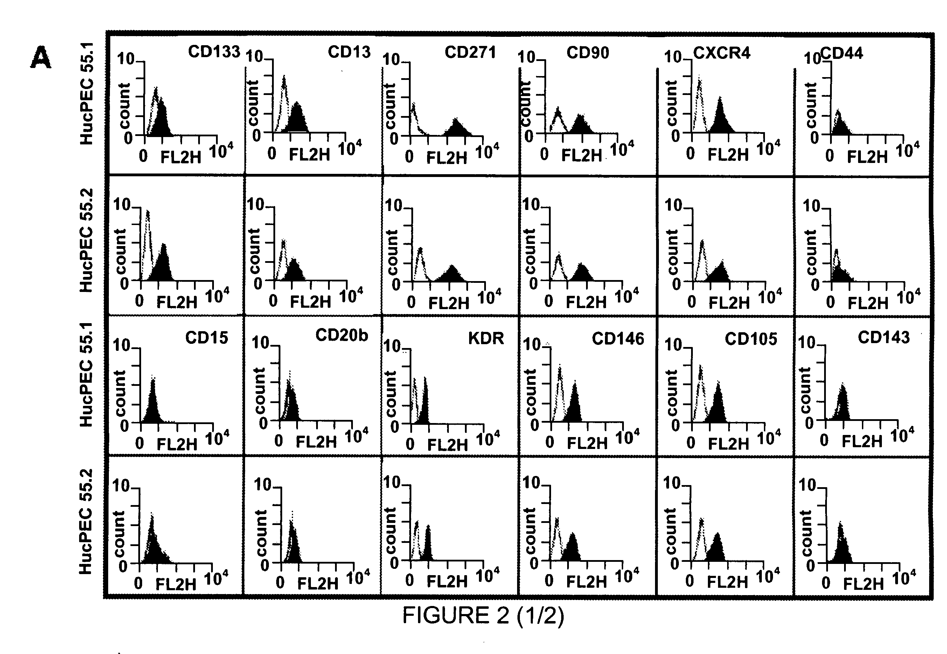

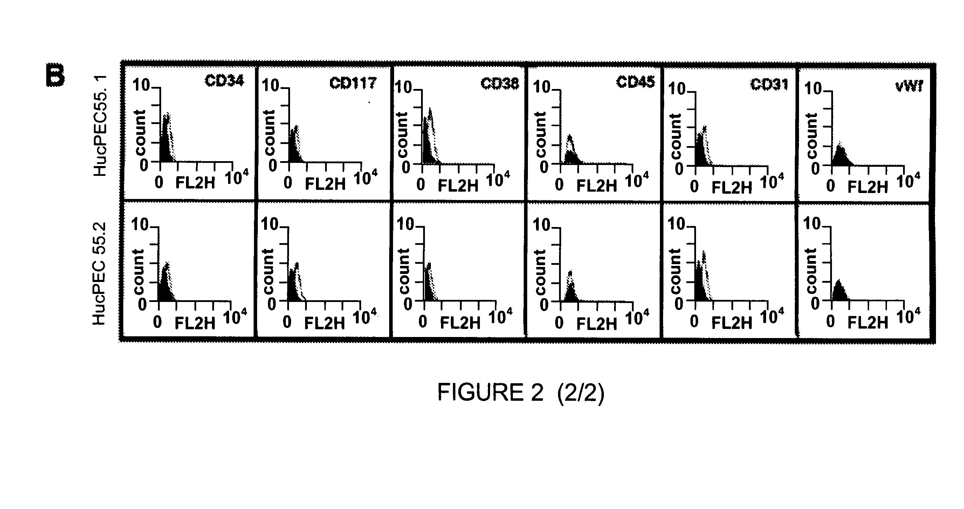

- La figure 2 représente des histogrammes de cytométrie en flux des lignées cellulaires HucPEC 55. 1 et HucPEC 55.2 avec des marqueurs positifs (A) et négatifs (B). Les courbes vides représentent les contrôles isotypiques et les courbes pleines les cellules marquées. L'ordonné représente le nombre de cellules (counts) et l'abscisse la fluorescense (FLH2).

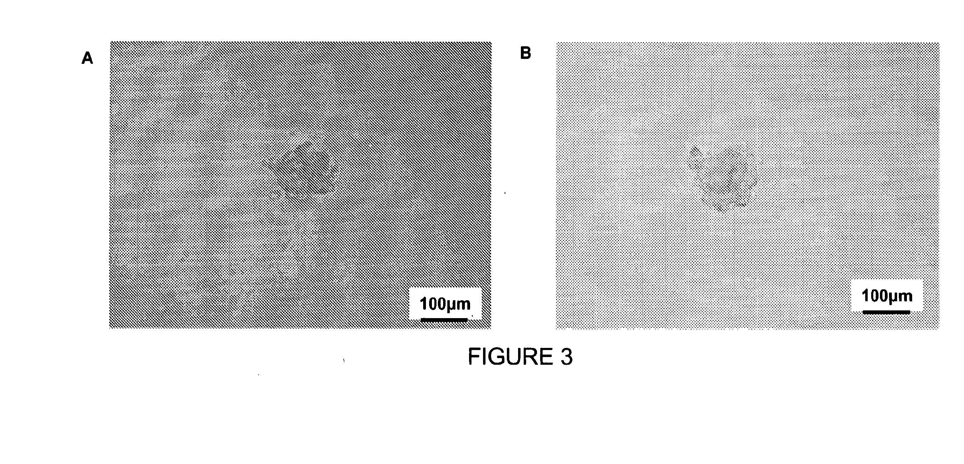

- La figure 3 est une photographie représentant la morphologie sphéroïde des lignées cellulaires HucPEC 55.1 et HucPEC 55.2 mises en culture dans un milieu de différentiation cellulaire hématopoïétique.

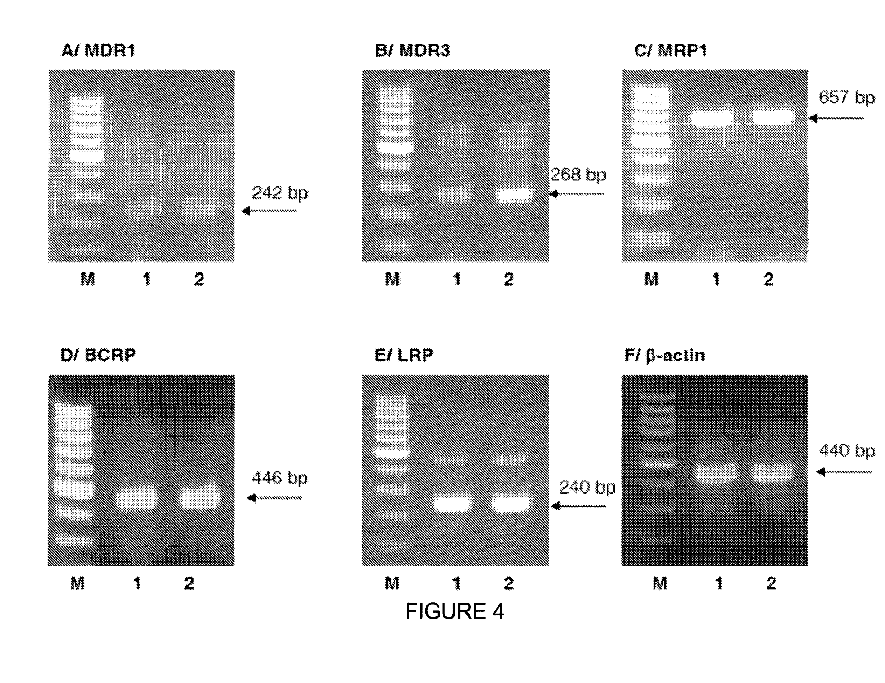

- La figure 4 représente des photographies de gels de transcrits d'ARNm codant pour des gènes de multi résistance aux médicaments (MDR) : La figure 4A représente une photographie dans lequel le transcrit code pour le MDR1 ; la figure 4B représente une photographie dans lequel le transcrit code pour le MDR3 ; la figure 4C représente une photographie dans lequel le transcrit code pour le MRP1 ; la figure 4D représente une photographie dans lequel le transcrit code pour le BCRP, la figure 4E représente une photographie dans lequel le transcrit code pour le LRP et la figure 4F représente une photographie dans lequel le transcrit est celui du gène de la β-actine. Sur les photographies, la ligne M correspond aux marqueurs de poids moléculaires; la ligne 1 aux cellules HucPEC 55.1; la ligne 2 aux cellules HucPEC 55.2.

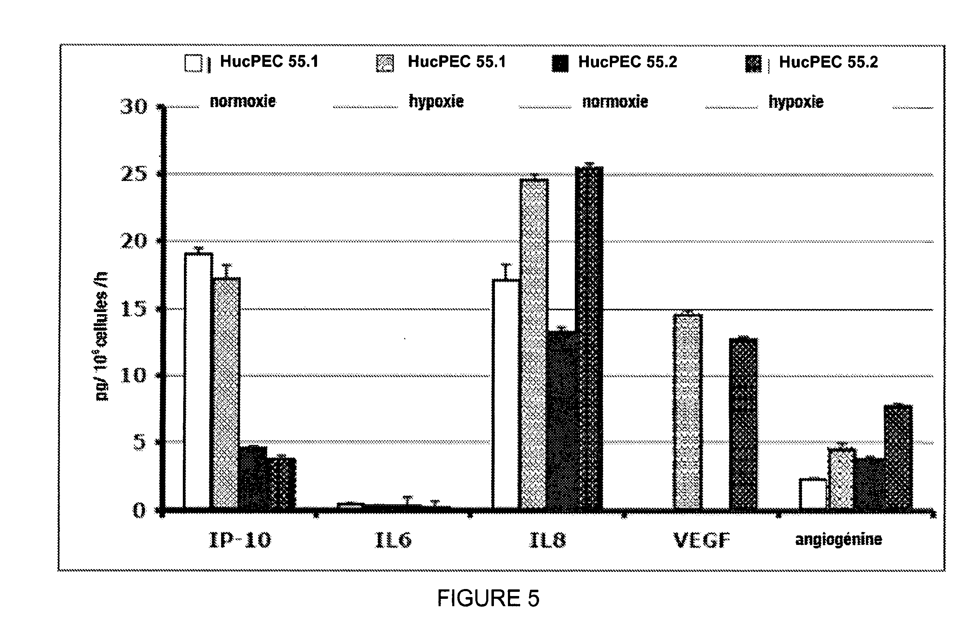

- La figure 5 représente un diagramme de la sécrétion de cytokines par les cellules HucPEC 55.1 et HucPEC 55.2 en conditions hypoxie ou normoxie. L'axe des ordonnées représente la quantité de facteurs sécrétés en fonction de la quantité de cellule et du temps et l'abscisse indique les différentes cytokines sécrétées.

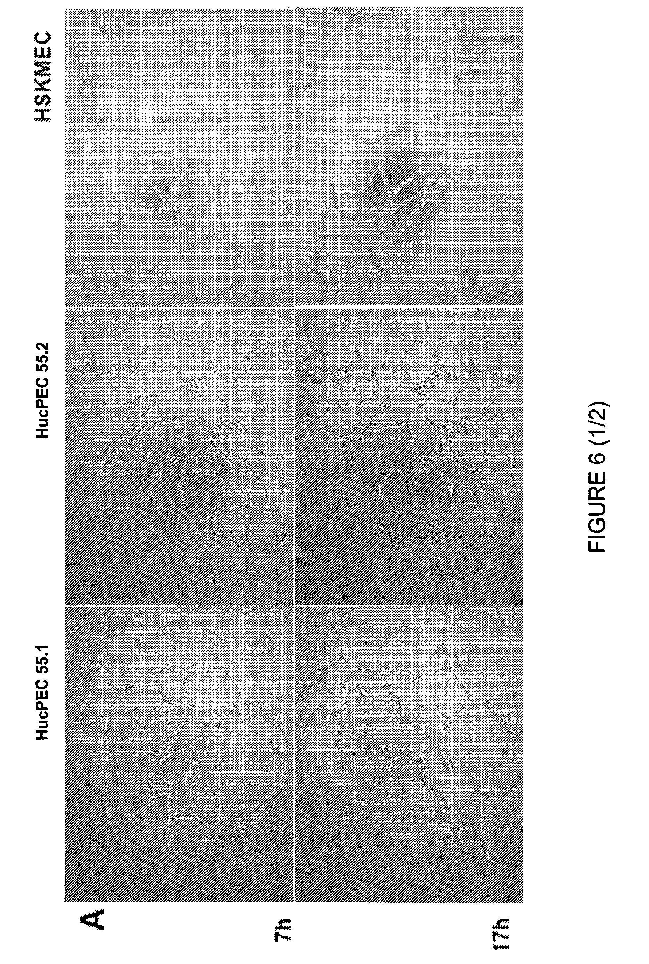

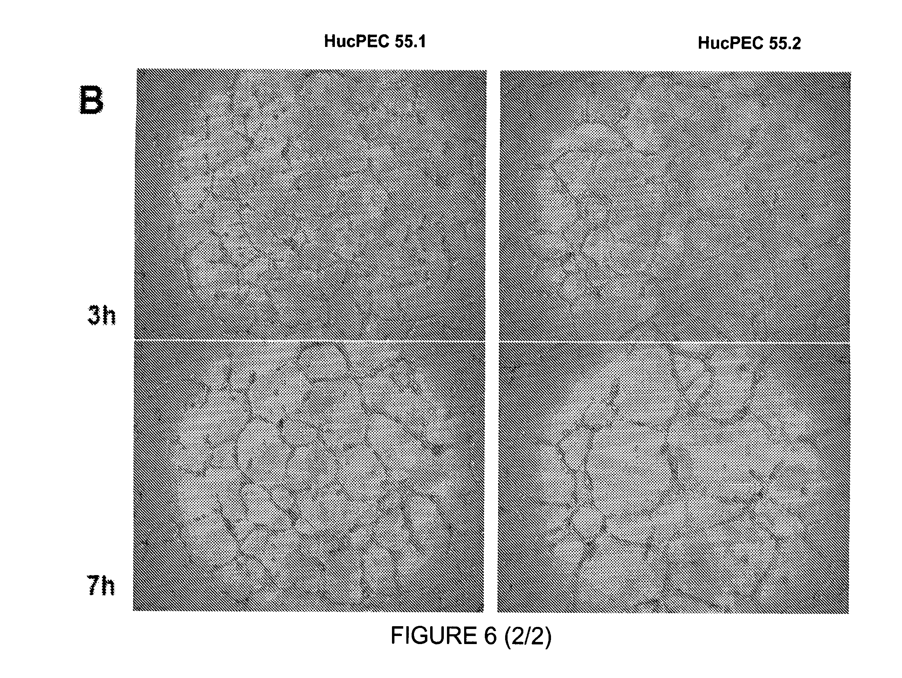

- Les figures 6 A et B représentent des photographies représentatives de la formation de pseudovaisseaux sur Matrigel (marque de commerce) en fonction du temps, par les cellules HucPEC 55.1, HucPEC 55.2 et HSkMEC.

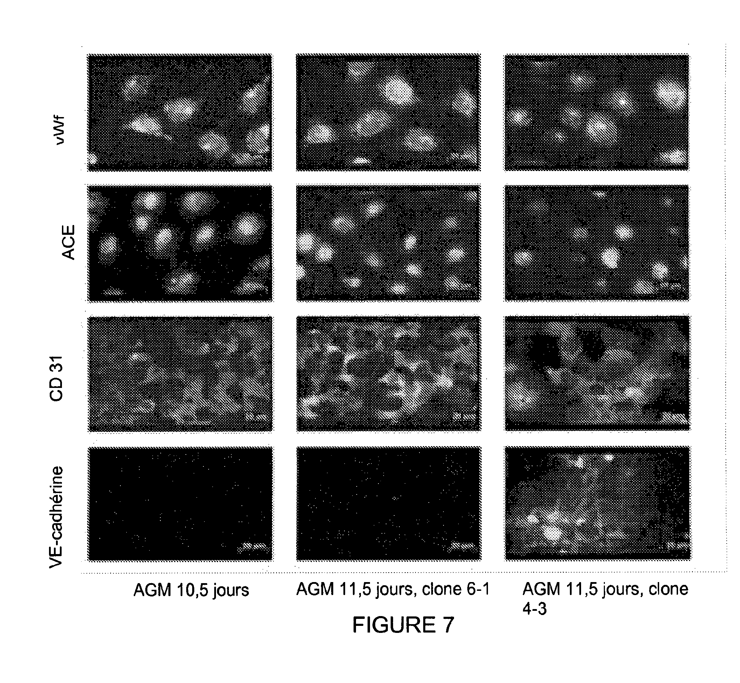

- La figure 7 représente des photographies de la détection de marqueurs spécifiques : la VE-cadhérine, le cluster de différentiation CD31, l'enzyme de conversion de l'Angitensine (ACE), le facteur vasculaire de von Willerbrand (vWf) en fonction des cellules précurseurs de cellules endothéliales félines et du temps AGM 10,5 jours, AGM 11,5 jours.

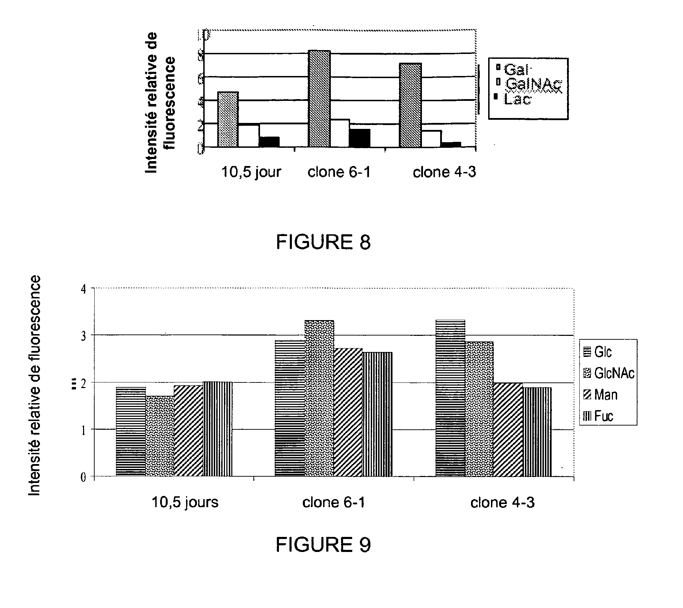

- La figure 8 est un diagramme en bâton représentant l'expression de galectines en fonction du temps. L'ordonnée représente l'intensité relative de la fluorescence correspondant au niveau d'expression et l'abscisse le temps.
- La figure 9 est un diagramme en bâton représentant l'interaction de Collectines avec des groupements contenant des résidus de type Glucose (Glc-), Glucose N acétyl (GlcNAc-), Mannose (Man-) et fucose (Fuc-). L'ordonnée représente l'intensité relative de la fluorescence correspondant au niveau d'expression et l'abscisse le temps.

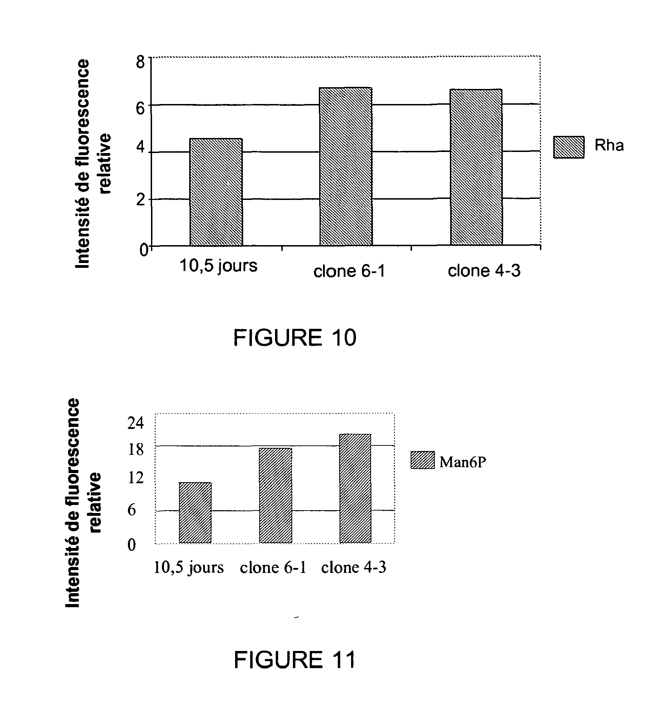

- La figure 10 est un diagramme en bâton représentant l'interaction de la lectine avec la rhamnose en fonction du temps. L'ordonnée représente l'intensité relative de la fluorescence correspondant au niveau d'expression et l'abscisse le temps.
- La figure 11 est un diagramme en bâton représentant l'expression du récepteur au Mannose 6Phosphate sur les cellules. L'ordonnée représente l'intensité relative de la fluorescence correspondant au niveau d'expression et l'abscisse le temps.

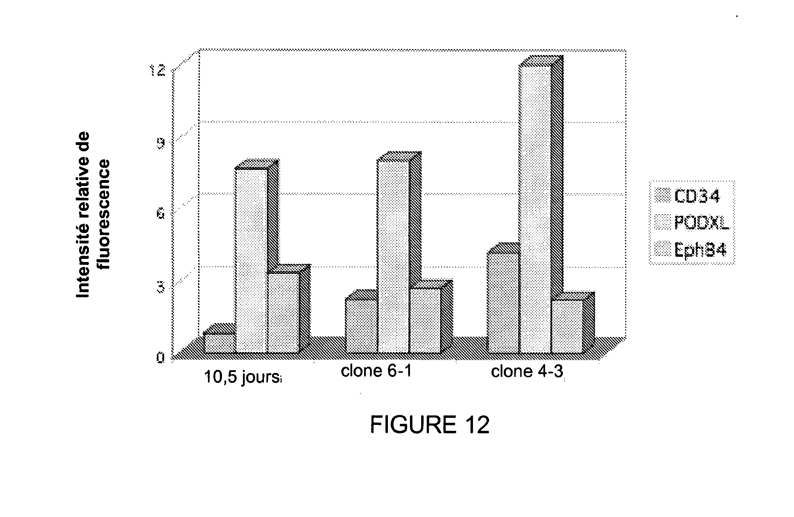

- La figure 12 est un diagramme en bâton représentant l'expression des marqueurs CD34, type podocalyxin (PODXL : « Podocalyxin-like ») et le récepteur Ephrine type B 4 (EphB4 : « Ephrin Type B Receptor 4 ») en fonction des cellules. L'ordonnée représente l'intensité relative de fluorescence et l'abscisse les cellules testées.

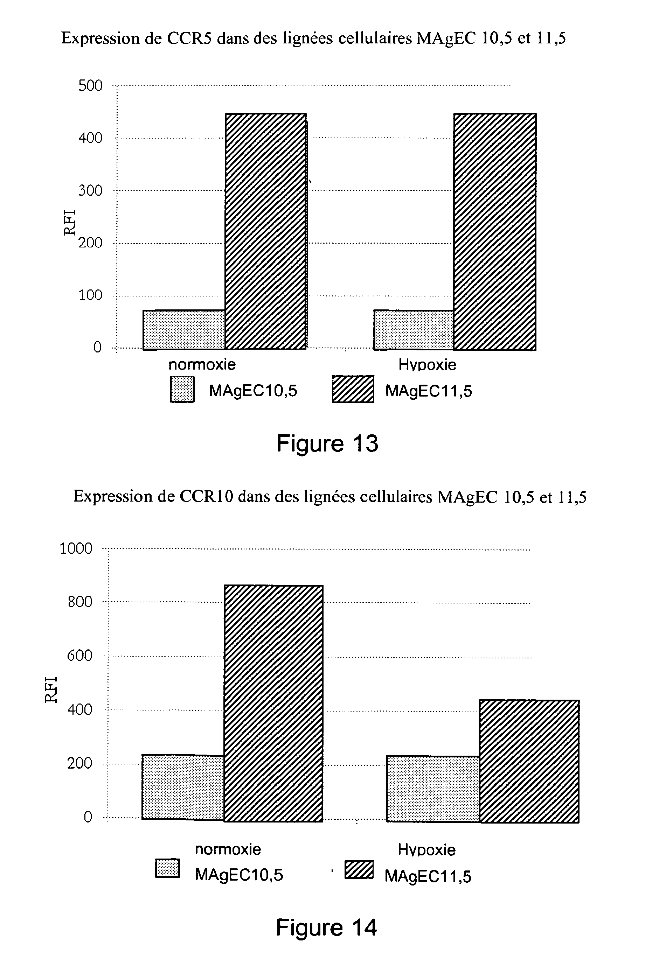

- La figure 13 est un diagramme en bâton représentant l'expression du marqueur CCR5 dans les lignées cellulaires MAgEC 10,5 (en pointillés) et 11,5 (hachuré) mesurée par cytofluorimétrie en flux en fonction des conditions de normoxie ou hypoxie. L'ordonnée représente l'intensité relative de fluorescence et l'abscisse les conditions normoxie ou hypoxie
- La figure 14 est un diagramme en bâton représentant l'expression du marqueur CCR10 dans les lignées cellulaires MAgEC 10,5 (en pointillés) et 11,5 (hachuré) en fonction des conditions de normoxie ou hypoxie, mesurée par cytofluorimétrie en flux. L'ordonnée représente l'intensité relative de fluorescence et l'abscisse les conditions normoxie ou hypoxie.

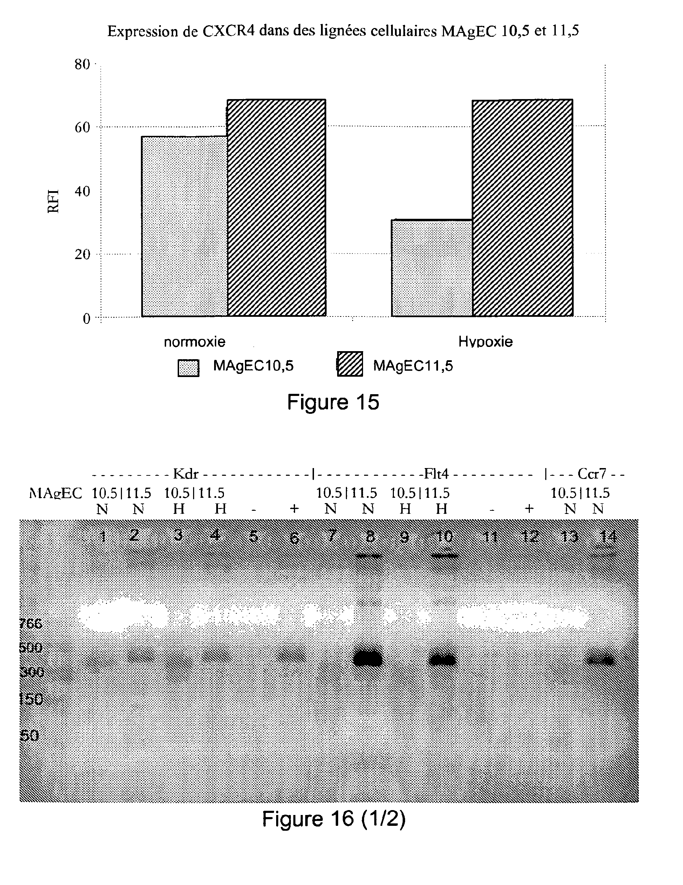

- La figure 15 est un diagramme en bâton représentant l'expression du marqueur CXCR4 dans les lignées cellulaires MAgEC 10,5 (en pointillés) et 11,5 (hachuré) mesurée par cytofluorimétrie en flux en fonction des conditions de normoxie ou hypoxie. L'ordonnée représente l'intensité relative de fluorescence et l'abscisse les conditions normoxie ou hypoxie.

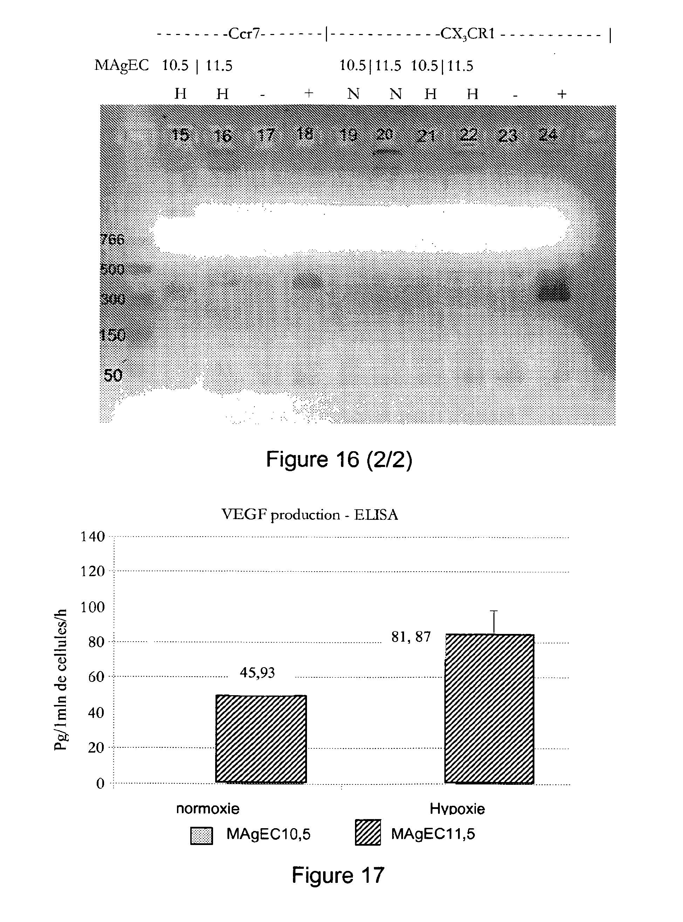

- La figure 16 représente des photographies de gels de transcrits d'ARNm codant pour les gènes Kdr (colonnes 1 à 4), Flt4 (colonnes 7 à 10), CCR7 (colonnes 13 à 16), CX₃CR1 (colonnes 19 à 22) dans les cellules MAgEC 10,5 et 11,5 en fonction des conditions de normoxie (N) ou hypoxie (H). Les colonnes - et + correspondent respectivement aux témoins négatifs et positifs.
- La figure 17 est un diagramme en bâton représentant la production de VEGF mesurée par ELISA par les lignées cellulaires MAgEC 10,5 (en pointillés) et 11,5 (hachuré) en fonction des conditions de normoxie ou hypoxie. L'ordonnée représente la quantité de VEGF estimée après établissement d'une courbe étalon, en pg (picogrammes) par million de cellules et l'abscisse les conditions normoxie ou hypoxie.

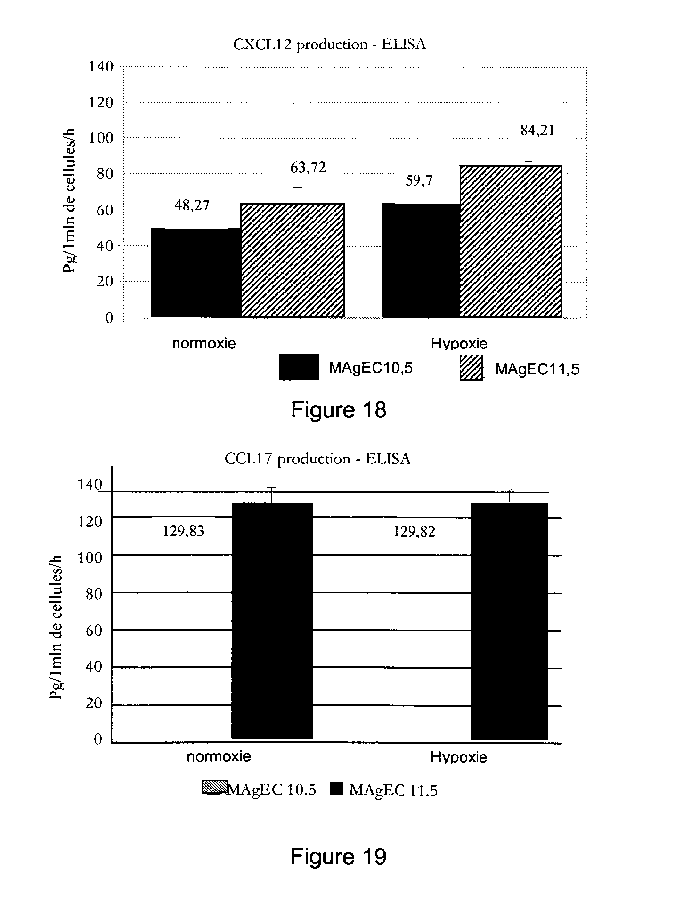

- La figure 18 est un diagramme en bâton représentant la production de CXCL12 mesurée par ELISA, par les lignées cellulaires MAgEC 10,5 (en noir) et 11,5 (hachuré) en fonction des conditions de normoxie ou hypoxie. L'ordonnée représente la quantité de VEGF estimé après établissement d'une courbe étalon, en pg (picogrammes) par million de cellules et l'abscisse les conditions normoxie ou hypoxie.
- La figure 19 est un diagramme en bâton représentant la production de CCL17 mesurée par ELISA par les lignées cellulaires MAgEC 10,5 (hachuré) et 11,5 (en noir) en fonction des conditions de normoxie ou hypoxie. L'ordonnée représente la quantité de VEGF estimé après établissement d'une courbe étalon, en pg (picogrammes) par million de cellules et l'abscisse les conditions normoxie ou hypoxie.

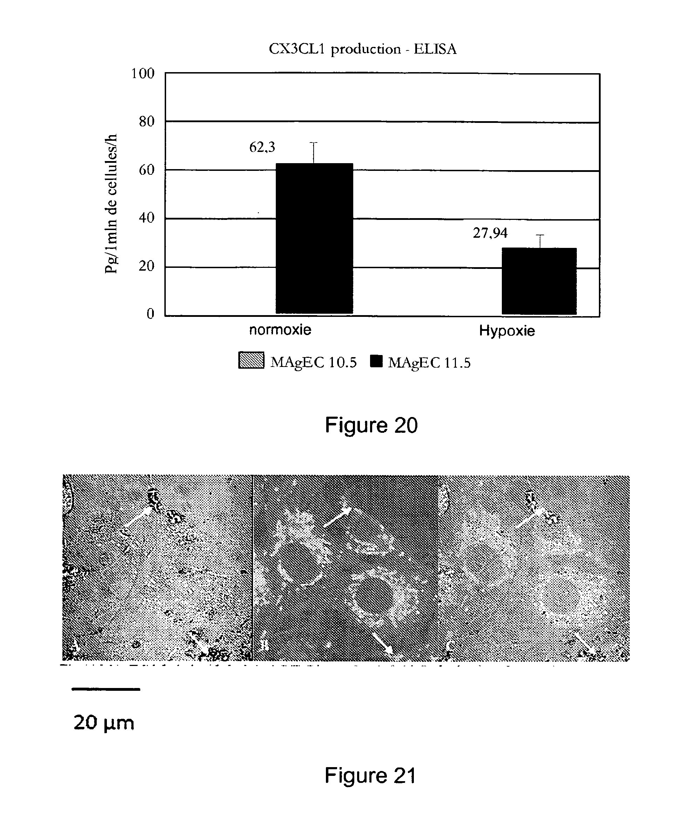

- La figure 20 est un diagramme en bâton représentant la production de CXCL1 visualisé par ELISA par les lignées cellulaires MAgEC 10,5 (hachuré) et 11,5 (en noir) en fonction des conditions de normoxie ou hypoxie. L'ordonnée représente la quantité de VEGF estimé après établissement d'une courbe étalon, en pg (picogrammes) par million de cellules et l'abscisse les conditions normoxie ou hypoxie.
- La figure 21 représente des photographies de cellules MAgEC 11.5 marquées avec 2mM de particules AMNP (Marquage par des nano-particules anioniques magnétiques) couplées avec la Rhodamine, A : sur fond clair, B : marquées avec la Rhodamine, C : sur fond clair et marquées.

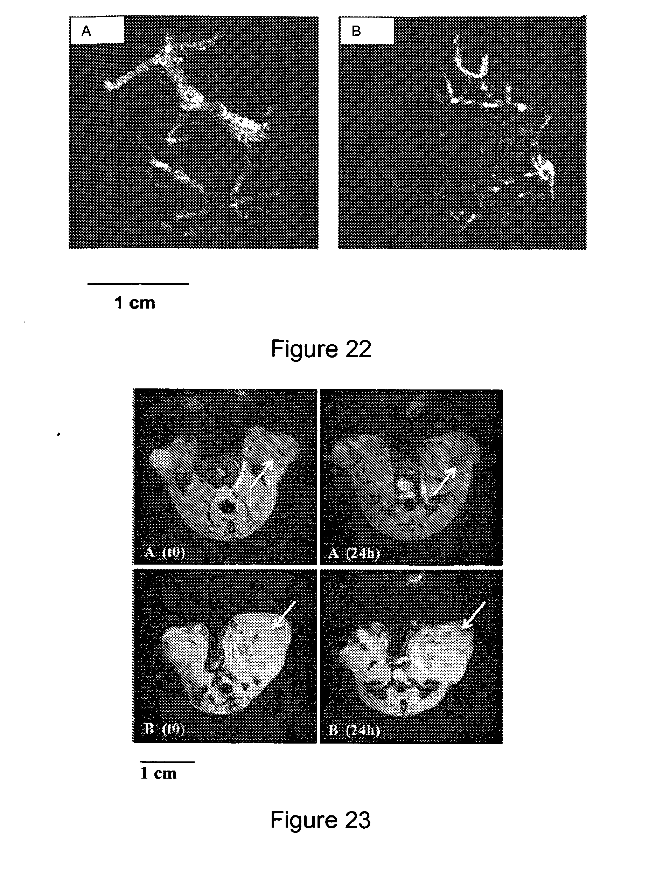

- La figure 22 représente des images obtenues par Imagerie par Résonance Magnétique de vaisseaux de muscles (A), de tumeurs sous-cutanées (B)
- La figure 23 représente des photographies de tumeurs sous cutanées B16(F10) obtenues par IRM de souris avant : A(t0) et 24 heures après injections intraveineuses de cellules MagEC 11.5 marquées A(24h), et des photographies de souris non injectées B(t0) et A(24h).

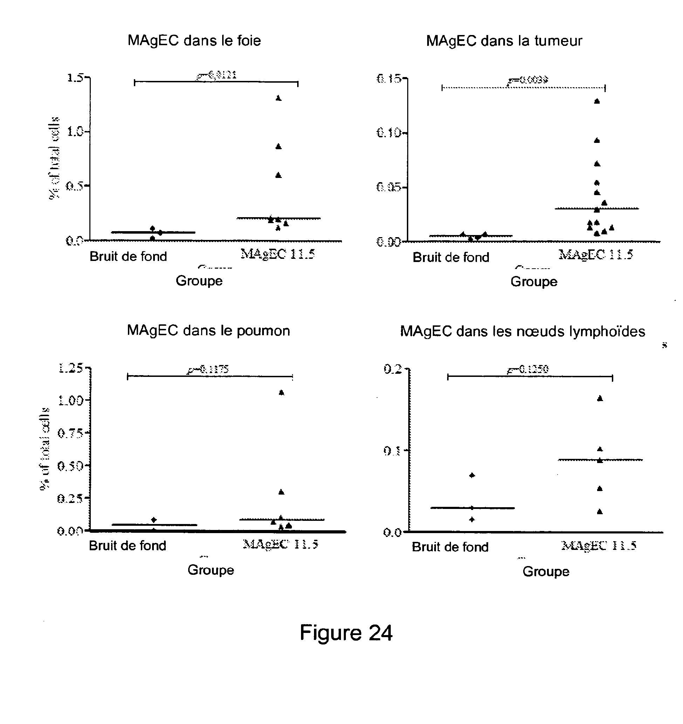

- La figure 24 représente des diagrammes représentant le pourcentage de cellules totales (abscisse) de MAgEC 11.5 chez une souris après injection de ces cellules en comparaison avec une souris sans injection (bruit de fond) dans la rate, dans une tumeur, dans les poumons et dans les ganglions lymphatiques

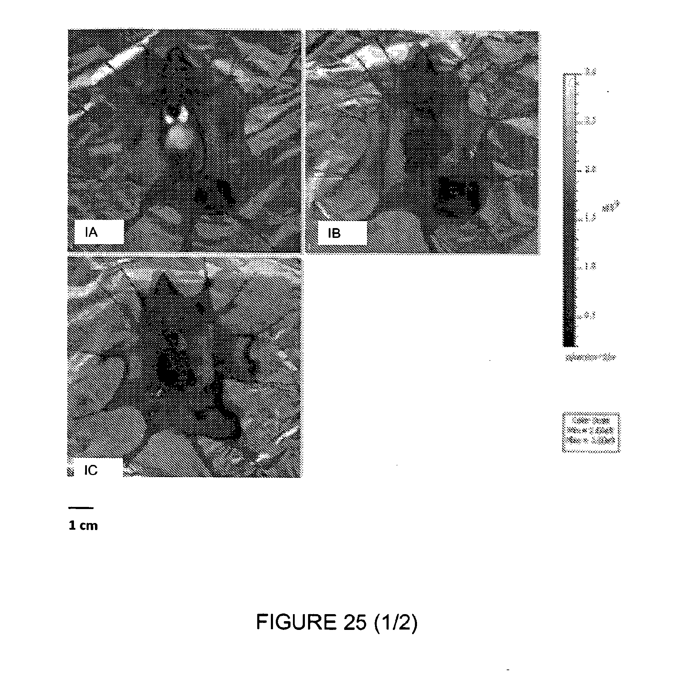

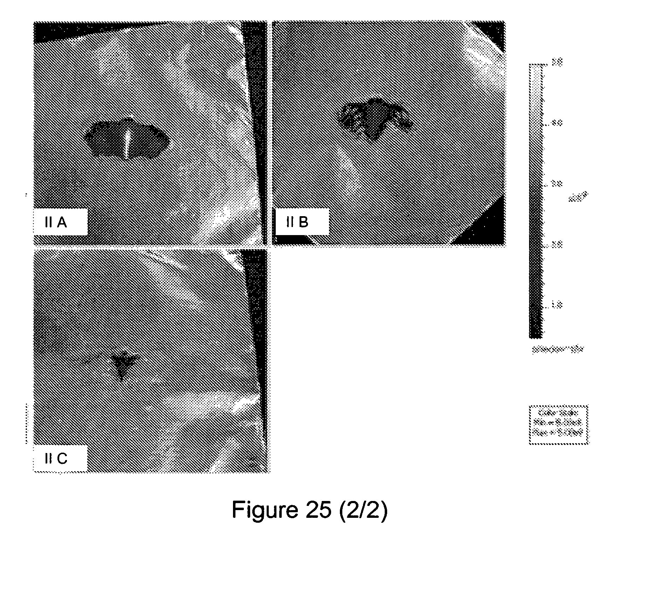

- La figure 25 représente des images obtenues par imagerie par fluorescence proche de l'infrarouge (« near infrared fluorescense imaging ») 7 jours après injection de cellules MAgEC 11.5. Les images IA, IB et IC sont des images du corps entier, les images IIA, IIB et IIC représentent les images de sternum et des os de la poitrine.

### EXEMPLES

### Exemple 1 : obtention et caractérisation des cellules humaines isolées précurseurs de cellules endothéliales

### A Matériels et Méthodes

### A.1 Echantillons sanguins

Des échantillons de sang ont été prélevés à partir de sang de cordons humains. Le sang du cordon a été collecté d'accouchements normaux, menés à terme au « Chair of Obstetrics and Gynecology » de l'université médicale de Wroclaw. La veine ombilicale a été piquée avec une aiguille de calibre 17 reliée à un système de sac de récupération fermé contenant une solution de dextrose de phosphate de citrate comme anticoagulant des sachets de sang (commercialisé par MACO Pharma, Wroc 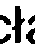 aw, Poland. Le volume récolté était en moyenne 50-110 ml à partir d'un placenta. Le consentement éclairé écrit a été obtenu de toutes les mères avant le travail et l'accouchement. Les protocoles d'échantillonnage du sang de cordon ombilical humain ont été approuvés par la Commission de Bioéthique à l'université médicale de Wroc 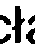 aw.

### A.2. Isolement de cellules mononucléaires.

Les cellules mononucléaires du sang de cordon ombilical ont été isolées par centrifugation de gradient de densité en utilisant Lymphoflot (marque de commerce) à une concentration de 1,077 g/ml (commercialisées par la société Biotest, Dreieich, Germany). La couche de cellules mononucléaires a été collectée, les cellules ont été lavées deux fois avec 1 mM d'éthylène diamine tetra acétate (EDTA) dans du tampon phosphate salin (« Phosphate Buffered Saline » PBS) et conservées congelées dans de l'azote liquide jusqu'à leur utilisation.

### A.3 Culture des cellules mononucléaires et leur immortalisation

Les cellules mononucléaires isolées (1 X 10⁶ ml) ont été cultivées dans des boîtes de culture de tissus dont le plastique est recouvert d'une couche de fibronectine humaine (Biocoat (marque de commerce) commercialisée par la société Becton Dickinson Biosciences, Grenoble, France) à 37°C dans une atmosphère de 95% air et 5% de CO₂. Le milieu utilisé pour la culture cellulaire était le EBM-2 (marque de commerce) commercialisé par la société Clonetics, Lonza, Saint Beauzire, France, supplémenté avec 10% de Sérum Foetal Bovin commercialisé par la société Hyclon, Longan, Utah, USA ; et des facteurs de croissance et de différentiation adaptés. Après 3 jours de culture, le milieu a été supplémenté avec le surnageant de cellules (50% vol/vol) de lignées cellulaires rétrovirales TE FLY GA hTERT obtenues dans notre laboratoire tel qu'indiqué dans Szyda A, Paprocka M, Krawczenko A, et al. Optimization of a retroviral vector for transduction of human CD34 positive cells. Acta Biochim Pol. 2006;53:815-823 [13].

Après 3 semaines de culture, 2 clones de cellules proliférantes ont été répliqués. Les cellules ont été maintenues 1 mois en culture dans du milieu EBM-2 medium, supplémenté avec 10% FCS (Hyclon) et des facteurs de croissance: rhSCF(Sigma), rhSCGF (100ng/mL), (AbD PeproTech, rhFlt-3 ligand (R&D systems, rh VEGF (R&D systems or Sigma.

Les deux clones obtenus ont été nommés HucPEC 55. 1 et HucPEC 55.2.

Des photos des cellules en croissance ont été prises avec le microscope Axiovert S Zeiss commercialisé par la société Jena, Allemagne équipé avec une caméra digitale Fine Pix 5602 (marque de commerce) commercialisée par la société Fuji Film.

### A.4. Immunomarquage des lignées cellulaires HucPEC 55.1 et HucPEC 55.2

Le phénotype des cellules cultivées a été analysé en utilisant des anticorps polyclonaux de lapin non couplés anti-facteur de Willebrand commercialisé par la société Sigma et les anticorps monoclonaux de souris suivants: anti-CD133 couplé à la phycoérythrine (PE) et anti-CD271 couplé à l'iso thiocyanate de fluorescéine (FITC) commercialisé par la société Miltenyi Biotec, Paris, France, anti-CD105 couplé au PE, anti-CD143, anti-CD146, anti-CXCR4, anti-hVEGFR2 et anti-CD202 non-couplés commercialisés par la société R&D Systems, anti-CD13 couplé au PE, anti-CD34, anti-CD38, anti-CD54, anti-CD90 commercialisé par la société Becton Dickinson Biosciences, anti-CD45 couplé au FITC, anti-CD44 et anti-CD15s non couplés commercialisé par la société Becton Dickinson Biosciences, anti-CD31 couplé au FITC commercialisé par la société Sigma.

Tous les anticorps directement marqués ont été utilisés a raison de 1 X 10⁵ cellules/échantillon, suspendues dans du tampon phosphate salin (« Phosphate Buffered Saline » PBS) supplémenté de 1% FCS à la concentration adaptée. L'incubation des cellules avec les anticorps se déroule pendant 30 min à 4°C. Après l'incubation, les cellules ont été soigneusement lavées. Les contrôles sont réalisés à l'aide d'immunoglobulines non spécifiques, de même isotype.

De manière similaire, trois anticorps non marqués ont été utilisés pour 1X10⁵ cellules/échantillon suspendues dans du PBS supplémenté de 1% FCS. L'incubation est réalisée pendant 30 min à 4°C. Après l'incubation, les cellules ont été soigneusement lavées et les anticorps secondaires anti-souris ou anti-lapin marqués au FITC ont été appliqués de manière subséquente pendant 30 min à 4°C.

Avant l'analyse par trieur de cellules Cytomètre en flux à fluorescence (« fluorescence-activated cell sorter » FACS), les cellules ont été lavées dans du PBS. Les contrôles sont réalisés à l'aide d'immunoglobulines de lapin ou de souris de même isotype.

Avant la détection du CD143 et du facteur von Willebrand, les cellules ont été fixées et perméabilisées avec 2% de paraformaldéhyde et 0,2% de saponine dans une solution de PBS pendant 10 min pour permettre le marquage intracellulaire.

Les cellules ont été analysées par cytométrie de flux en utilisant FACSCalibur (marque de commerce) commercialisé par la société Becton Dickinson, CA, USA. Les données ont été acquises et enregistrées pour 5000-10000 événements en utilisant CellQuest software (marque de commerce) commercialisé par la société Becton Dickinson et présentées pour être analysées sous forme d'histogrammes en utilisant le logiciel WINMDI 2.7.

### A.5. Capacité des lignées cellulaires HucPEC 55. 1 et HucPEC 55. 2 à croître et se différencier en cellules hématopoïétiques dans un milieu semi-sol ide

La capacité des cellules HucPEC 55. 1 et HucPEC 55. 2 à croître et se différencier en cellules hématopoïétiques a été évaluée en utilisant MethoCult GF H4434 (marque de commerce) commercialisé par la société StemCell Technologies Inc.Vancouver, Canada, contenant les cytokines recombinantes suivantes : rhSCF (50 ng/ml), rhGM-CSF (10 ng/ml), rhIL3 (10 ng/ml) et érythropoïétine recombinante (3 unités/ml).

10³ cellules des deux lignées cellulaires testées ont été suspendues dans 1ml du milieu semi-solide MethoCult (marque de commerce) commercialisé par la société StemCell et transférées dans des plaques de 24 puits. Après 7 jours de culture, des photos des clusters de cellules en croissance ont été prises.

### A.6 Détection des ARNm dans les précurseurs cellulaires des lignées HucPEC 55. 1 et HucPEC 55. 2 par transcription réverse et réaction de polymérase en chaîne (RT-PCR)

L'ARN cellulaire total a été isolé de 2.5 X 10⁶ cellules en utilisant le MiniKit RNeasy Protect (marque de commerce) commercialisé par la société Qiagen, Courtaboeuf, France. Le premier brin d'ADNc synthétisé a été fait par transcription réverse de 1 µg de l'ARN total en utilisant l'Omniscript Reverse Transcriptase (marque de commerce) commercialisé par la société Qiagen. La réaction de réaction en chaîne par polymérase (PCR) pour détecter les ARNm des MDR1, MDR3, MRP1, BCRP et LRP ainsi que de la β actine (gène gardien) a été faite en utilisant des amorces spécifiques et les conditions de réalisations décrites dans le Tableau 1.

**Tableau 1**

| Amorces | SEQ ID NO | Séquence nucléotidique | Conditions de réalisation de la PCR |
|---|---|---|---|
| MDR-1s | 1 | 5'-AAGCTTAGTACCAAAGAGGCTCTG-3' | 37 cycles de 94°C, 1 min; 58°C, 1 min; 72°C, 1 min produit obtenu: 243 pdb |
| MDR-1as | 2 | 5'-GGCTAGAAACAATAGTGAAAACAA-3' | |
| MDR3-s | 3 | 5'-AGGGCGACTTTGAACTGGGC-3' | 35 cycles de [94°C, 1 min; 60°C, 1 min; 72°C, 2 min], produit obtenu: 268 pdb |
| MDR3-as | 4 | 5'-TTTGCCTGGATTTAGCAGCG-3' | |
| MRP1-s | 5 | 5'-AGTGACCTCTGGTCCTTAAACAAGG-3 | 35 cycles de [94°C, 1 min; 56°C, 1 min; 68°C, 1 min], produit obtenu: 657 pdb |
| MRP1-as | 6 | 5'-GAGGTAGAGAGCAAGGATGACTTGC- 3' | |
| BCRP-s | 7 | 5'-TTAGGATTGAAGCCAAAGG-3' | 35 cycles de [94°C, 40s₋51°C 1min: 72°C, 1 min], produit obtenu: 446 pdb |
| BCRP-as | 8 | 5'-TAGGCAATTGTGAGGAAAATA-3' | |
| LRP-s | 9 | 5'-GTCTTCGGGCCTGAGCTGGTGTCG-3' | 33 cycles de [94°C, 45 s; 55°C, 45 s;72°C, 45 s], produit obtenu: 240 pdb |
| LRP-as | 10 | 5'-CTTGGCCGTCTCTTGGGGGTCCTT-3' | |
| β actine-s | 11 | 5'-CCAGAGCAAGAGAGGCATCC-3' | 28 cycles de [94°C, 30 s; 55°C, 45 s; 72°C, 60 s], produit obtenu: 450 pdb |
| P actine-as | 12 | 5'-CTGTGGTGGTGAAGCTGAAG-3' | |

### A.7. Sécrétion de diverses cytokines par les lignées cellulaires HucPEC 55.1 et HucPEC 55.2

La sécrétion des cytokines a été évaluée en utilisant le test de billes cytométriques commercialisé par la société Becton Dickinson Biosciences. Les molécules suivantes ont été testées : protéine inductible par l'interféron 10 (interferon inductibe protein 10, IP-10), interleukine 6 (IL6), interleukine 8 (IL8), Facteur de croissance de cellules endothéliales vasculaires (VEGF), angiogénine.

Les surnageants des lignées cellulaires HucPEC 55. 1 et HucPEC 55. 2 ont été collectés après culture pendant 16h soit en normoxie (20% 02) soit en hypoxie (1% 02) et conservés congelés à - 80°C avant de les utiliser.

Les expériences ont été faites en accord avec le protocole du fabricant. Environ 300 billes ont été analysées pour chaque cytokine. Les analyses de cytométrie de flux du marquage des billes ont été réalisées en utilisant le LSR (marque de commerce) commercialisé par la société Becton Dickinson et les résultats ont été analysés en utilisant le logiciel de test FCAP (marque de commerce) commercialisé par la société Becton Dickinson.

### A. 8. Formation de pseudovaisseaux.

La matrice Matrigel (marque de commerce) commercialisé par la société Becton Dickinson Biosciences a été diluée à 1/2 dans le milieu basal OptiMEM (marque de commerce) commercialisé par la société Invitrogen, Cergy Pontoise, France, à 4°C, distribué dans des microplaques de 96 puits dans un volume de 50 µl et laissées à polymériser à 37°C pendant 30 min. Les cellules (1,2 X 10⁴ cellules) des lignées HSkMEC (cellules humaines microvasculaires de peau) obtenues selon le procédé décrit dans Kieda et al. « New human microvascular endothelial cell lines with specific adhésion molecules phenotypes. » Endothelium. 2002;9(4):247-61 [**14**]. Les cellules HucPEC 55. 1 et HucPEC 55. 2 ont été ensemencées sur des microplaques revêtues de Matrigel (marque de commerce) commercialisé par la société Becton Dickinson Biosciences dans un volume de 100 µl et les cultures ont été faites soit dans des conditions de normoxie (20% O₂) soit dans des conditions d'hypoxie (1% O₂). Les photos ont été prises à l'aide d'un microscope inversé (Axiovert 200M (marque de commerce) Zeiss, Le Pecq, France) équipé d'une caméra numérique à haute résolution Axiocam (marque de commerce) commercialisée par la société Zeiss, Le Pecq, France) reliée à un ordinateur exécutant le logiciel d'acquisition Axiovision commercialisé par la société Zeiss. La visualisation directe et en temps réel de la formation des pseudo-vaisseaux a été surveillée pendant 24h.

### B. Résultats

Deux clones de cellules en prolifération ont été obtenus après la transduction des cellules mononucléaires sanguines du cordon ombilical humain avec un surnageant de la lignée cellulaire TE FLY GA hTERT [13], produisant des rétrovirus contenant le gène de la transcriptase inverse des télomérases humains. Les transductions ont été faites sur 4 échantillons différents de cellules mononuclées sanguines de cordon ombilical, cultivées dans les mêmes conditions, en présence d'un mélange de facteurs promoteurs de la croissance. Deux clones ont été obtenus et nommés HucPEC 55. 1 et HucPEC 55. 2 La morphologie des deux lignées cellulaires est représentée sur la Figure 1

### B.1. Détermination de l'immunophénotype des deux lignées cellulaires HucPEC 55. 1 et HucPEC 55. 2

Les deux lignées cellulaires ont tout d'abord été analysées pour l'expression des marqueurs des cellules précurseurs/cellules souche et pour les marqueurs des cellules endothéliales, comprenant des récepteurs de facteurs de croissance et des molécules d'adhésion choisies comme présenté sur la Figure 2A. Les deux lignées cellulaires expriment CD133 qui est connu pour être un marqueur général des cellules souches normales et néoplasiques. Elles expriment également CD13 (marqueur des cellules souches hématopoïétiques), CD271 (marqueur des cellules souches non hématopoïétiques) et CD90 (marqueur des cellules souches mésenchymateuses). Les deux lignées cellulaires étaient positives pour l'expression de CXCR4 et faiblement positives pour CD44 et CD15s, qui peuvent être exprimées à la fois sur les cellules souches et sur les cellules endothéliales. Les cellules expriment également des marqueurs typiques des cellules endothéliales : CD202b, VEGFR2, CD146, CD105 et expriment faiblement CD143. Les marqueurs qui ne sont pas exprimés par ces lignées cellulaires sont rassemblés comme présenté sur la Figure 2B. Les deux lignées HucPEC 55.1 et HucPEC 55.2 étaient négatives pour CD34, CD117, CD38 et CD45 ce qui suggère qu'elles ne sont pas d'origine hématopoïétique. Elles n'exprimaient pas les CD31 et vWf, marqueurs des cellules endothéliales différenciées.

### B.2. Capacité des lignées cellulaires HucPEC 55.1 et HucPEC 55.2 de croissance et de différentiation en cellules hématopoïétiques dans un milieu semi-solide.

Les deux lignées cellulaires ont été cultivées dans un milieu semi-solide supplémenté en facteurs de croissance et de différentiation hématopoïétiques pour évaluer leur capacité à se différencier en cellules hématopoïétiques. Les deux lignées cellulaires sont capables de croître dans un milieu semi-solide, mais ne se différencient pas en cellules hématopoïétiques. Après 7 à 9 jours de culture, les cellules forment des sphéroïdes multicellulaires compacts. Elles ne montrent aucun signe de différentiation en érythrocytes, granulocytes ou monocytes, comme le montre la Figure 3.

Cette expérience démontre que les cellules isolées ne sont donc pas des cellules précurseurs de cellules hématopoïétiques.

### B.3. Expression des transcrits des ARNm de résistance multidrogues détectés par RT-PCR

Une réverse transcriptase de la réaction en chaîne par polymérase (RT-PCR) des ARNm codant pour les protéines de résistance multidrogues a été utilisée pour suivre l'expression de leurs gènes dans les lignées HucPEC 55. 1 et HucPEC 55. 2. L'expression des produits des gènes pour MDR1, MDR3, MRP1, BCRP et LR est présentée sur la Figure 4. Les ARNm de toutes les protéines de multi résistance aux drogues sont présents dans les deux lignées cellulaires avec des niveaux similaires, à l'exception de MDR3 qui semble être exprimée à un niveau plus élevé dans la lignée HucPEC 55. 2 comparé à la lignée HucPEC 55.1.

### B.4. Sécrétion de cytokines par les lignées cellulaires HucPEC 55. 1 et HucPEC 55. 2

Le test cytométrique sur billes a été utilisé pour évaluer la sécrétion des cytokines choisies (IP-10, IL6, IL8, VEGF et angiogénine) par les cellules HucPEC 55. 1 et HucPEC 55. 2 en croissance dans des conditions normoxiques et hypoxiques. La Figure 5 montre que l'altération de la pression partielle d'oxygène influence la production de cytokines proangiogéniques à des points variables par les deux lignées cellulaires. En effet, la production de cytokines proangiogéniques IL8 et angiogénine a augmenté de manière significative en hypoxie. Les deux lignées cellulaires n'ont pas produit du VEGF en normoxie, mais l'expression de ce facteur proangiogénique a été induite par l'hypoxie dans les deux lignées cellulaires. IP10, qui est une cytokine antiangiogénique, a été produite à des niveaux beaucoup plus élevés par HucPEC 55. 1 comparé à HucPEC 55. 2 et n'a pas été changé par l'hypoxie.

### B.5. Propriétés angiogéniques des lignées cellulaires HucPEC 55. 1 et HucPEC 55. 2

Les deux lignées cellulaires ont été testées pour leur potentiel angiogénique et le réarrangement en pseudo vaisseaux sur une matrice semi solide. La cinétique de ce réarrangement vers des structures de type tube a été comparée en hypoxie (1% 02) versus normoxie. La Figure 6A indique qu'en normoxie, les deux lignées HucPEC 55. 1 et HucPEC 55. 2 sont capables de commencer le processus angiogénique d'une manière comparable à la lignée de référence HSkMEC qui est une lignée cellulaire microvasculaire endothéliale différenciée et dérivée de la peau. Ce résultat démontre clairement que les cellules se sont connectées ensemble pour former un réseau. Le réseau a évolué pendant moins de 7 heures, pour les lignées cellulaires HucPEC 55. 1 et HucPEC 55. 2, puis elle s'est arrêtée. Concernant la lignée HSkMECs, son évolution a duré 17 heures.

Après 7 heures, les lignées cellulaires HucPEC 55. 1 et HucPEC 55. 2 sont restées où elles étaient et ont commencé à proliférer. Les conditions hypoxiques ont accéléré de manière efficace le processus de formation du tube pour les cellules HucPEC 55. 2, comme cela est montré sur la Figure 6B. Ce processus a aussi été accéléré pour la lignée HucPEC 55. 1 bien qu'il soit resté plus lent que pour HucPEC 55. 2. Outre l'accélération, l'hypoxie a causé également une différentiation plus complète vers la formation de structures de type vaisseaux qui n'étaient pas accomplie en normoxie par aucune des lignées cellulaires HucPEC 55. 1 et HucPEC 55. 1 et HucPEC 55. 2.

Tel que démontré dans cet exemple les lignées cellulaires établies HucPEC 55. 1 et HucPEC 55. 2 sont des cellules précurseurs de cellules endothéliales, qui ont des propriétés angiogéniques et ont un temps d'évolution inférieur aux lignées cellulaires connues permettant une prolifération plus rapide.

### Exemple 2 : obtention et caractérisation des cellules murines isolées précurseurs de cellules endothéliales

### A Matériels et Méthodes

### A1. Isolement des cellules

Les cellules ont été isolées à partir de la région Aorto-Gonado-Mésonéphrotique (AGM pour « Aorta Gonad Mesonephros régions ») d'embryons C57BI6 10,5 et 11, 5 jours post *coitum* (dpc pour « day post *coitum* »).

Les embryons ont été obtenus à partir de croissement de souris FVB provenant du laboratoire Transgénèse et Archivage d'Animaux Modèles - UPS44 (TAAM) CNRS Orléans.

Après une culture primaire sur des plaques primaria (Falcon, Becton and Dickinson) dans du RPMI (« Roswell Park Memorial Institute médium »), les cellules ont été laissées pour croissance et les cultures résultants ont été immortalisées tel que décrit précédemment pour l'immortalisation des cellules humaines après 4 jours via l'utilisation d'un plasmide contenant l'antigène large T SV40 large et des liposomes cationiques.

Des clones génétycines résistants ont été obtenus et sélectionnés. Différents clones ont été développés et caractérisés sur la base de l'expression de marqueurs de cellules endothéliales.

A partir de ces clones, deux lignées immortalisées ont été obtenues et utilisées pour des expériences complémentaires, il s'agit de :
- MAgEC 10,5: pour cellules endothéliales d'embryons de souris Gonado aortique mésonéphros 10,5 jour post *coitum* (« Mouse Aorta-Gonad Mesonephros Endothelial Cells » SV40 large T-antigen immortalization)
- MAgEC 11,5 : pour cellules endothéliales d'embryons de souris Gonado aortique mésonéphros 11, 5 jour post *coitum* (« Mouse Aorta-Gonad Mesonephros Endothelial Cells » (SV40 large T-antigen immortalization))

Les cellules ont été cultivées dans un incubateur à 37°C avec une atmosphère humidifiée comprenant 5% CO₂, 21% O₂.

Toutes les lignées cellulaires ont été cultivées dans un milieu OptiMEM medium (commercialisé par Gibco, France) complété avec 2% de FBS (commercialisé par PAA, Austria), 0,2% de fungizone (250 mg/ml, commercialisé par Gibco, France) et 1% de pénicilline-streptomycine (10.000 unités/ml, commercialisé par Gibco, France)

### A.2 Caractérisation des cellules

### A.2.1. Analyse de l'expression de gènes par transcription réverse et réaction de polymérase en chaîne (RT-PCR)

L'ARN total a été isolé avec un kit Qiagen RNeasy Kit (marque déposée) selon le protocole du fournisseur. 750 ng ou 1000 ng ont été chargés pour une réaction standard de transcription inverse en utilisant des amorces oligo-dT.

L'expression de différents gènes a été observée, en particulier les gènes codant pour la chimiokine (C-X-C motif) ligand 16 (CXCL16), le gène SDF-1, le gène codant pour la chimiokine (C-X3-C motif) ligand 1 (CX3CL1), le gène codant pour la chimiokine 6 cystéine (6CKine), le gène CTACK, le gène codant pour le récepteur du domaine d'insertion de la kinase ((Kdr) Kinase insert domain receptor), le gène codant pour le récepteur tyrosine kinase pour les facteurs de croissances endothéliales c et d ((Flt4) tyrosine kinase receptor for vascular endothelial growth factors C et d »), le gène codant pour le récepteur de la chimiokine (CC motif) (CCR7), le gène codant pour la chimiokine (C-X3-C motif) récepteur 1 (CXC₃CR1). Les Réactions de Polymérisation en Chaine (PCRs) ont été réalisées en utilisant les amorces décrites dans le tableau 2 et avec le protocole (a) à (e) suivant :

(a) 95⁰C – 3 min
(b) 95⁰C – 45 sec
(c)Température d'hybridation – 45 sec
(d) 72⁰C – 45 sec
(e) 72⁰C – 7 min
(b), (c) et (d) répétées 30 fois (d)
(30 cycles)

**Tableau 2**

| Gène | SEQ ID NO | amorce | Séquence nucléotidique |
|---|---|---|---|
| CXCL16 | 13 | Sens | 5'-AGACAGCAAGAAGCACCAGG-3 |
| | 14 | Anti-sens | 5'- CCTCTCCCATGTCATCATCC-3' |
| SDF-1 | 15 | Sens | 5'-GTTCTTCGAGAGCCACATCG-3' |
| | 16 | Antisens | 5'-ATGGCGGAGTGTCTTTATGC-3' |
| CX3CL1 | 17 | Sens | 5'-TGCGACAAGATGACCTCACG-3 |
| | 18 | Antisens | 5'-ATCCTGTGCCTCGGAAGTTG-3' |
| 6CKine | 19 | Sens | 5'-GGACTGCTGCCTTAAGTACAGC-3' |
| | 20 | Antisens | 5'-GCTATCCTCTTGAGGGCTGTG-3' |
| CTACK | 21 | Sens | 5'-GGCTGAGTGAGTGAGCATGATGG-3' |
| | 22 | Antisens | 5'-GGGATGAACACAGACACTGC-3' |
| Kdr | 23 | Sens | 5'-AGAGTGTGTCCCTGTTGTGC-3' |
| | 24 | Antisens | 5'-GGGGTAGGATTTCCAGATGC-3' |
| Flt4 | 25 | Sens | 5'-GAGTGACTCCCTGGAGATGC-3' |
| | 26 | Antisens | 5'-TAGCCCGTCTTGATGTCTGC-3' |
| Ccr 7 | 27 | Sens | 5'-AAACCCAGGAAAAACGTGC-3' |
| | 28 | Antisens | 5'-RGTAGACGCCAAAGATCCAGG-3' |
| CXC3R1 | 29 | Sens | 5'-TCTTCATCACCGTCATCAGC-3' |
| | 30 | Antisens | 5'-GATGCGGAAGTAGCAAAAGC-3' |

La température d'hybridation des amorces décrites dans le tableau 2 était de 57°C.

Les produits issus de cette RT-PCR ont été déposé dans un gel de migration afin d'identifier si le gène est exprimé.

### Test ELISA

Tout les tests ELISA ont été réalisés avec le système R&D ELISA DuoSet (marque déposée) selon le protocole du fabriquant. Après collecte des surnageants cellulaires, ils ont été comptés et les résultats ont été normalisés aux nombres de cellules.

La détection du facteur de von Willebrand exprimé dans le cytoplasme, dans les plaques de Weibel Palade, de l'enzyme de conversion de l'Angiotensine présente dans la membrane nucléaire, le cluster de différentiation CD31 et la VE-cadhérine a été réalisée par microscopie de fluorescence et en contraste de phase équipé avec le DIC (Zeiss, Axiovert 200).

### A.2.3. Sécrétion de cytokines par les lignées cellulaires

Le procédé CBA (« Cytometric Bead Array ») commercialisé par la société Becton-Dickinson, USA, le cytomètre en flux LSR commercialisé par BD, USA et le logiciel FCAP Array Software commercialisé par BD, USA ont été utilisés pour déterminer les niveaux de KC, TNFα, IFNγ, GM-CSF, IL-1β, IL-4, IL-6, IL-10, IL-12 collectés dans le surnageant selon le protocole du fournisseur. Cinq courbes paramétriques logistiques ont été ajustées à des échantillons standards et les résultats ont été normalisées au nombre de cellules.

La production de VEGF, des chimiokines CXCL12, CCL17 et CXCL1 par les lignées cellulaires MAgEC 10,5 et 11,5 a également été étudiée.

### A.2.4. Immunomarquage des lignées cellulaires

Le cytomètre en flux LSR commercialisé par BD a été utilisé pour déterminer la fluorescence et le marquage cellulaire. Le logiciel FlowJo v.5.7.2 a été utilisé pour analyser les résultats.

Pour le marquage, les cellules ont été lavées avec une solution de cPBS/0,5% BSA/ 0,1% sodium azide, détaché avec 1 mg/mL de collagénase type I commercialisé par la société Gibco, Invitrogen, fixé et perméabilisé avec du Cytofix/Cytoperm commercialisé par BD, USA selon le protocole du fournisseur. 5 x 10⁵ cellules ont été prises par tube et marquées avec un anticorps primaire dans un volume de 30µl de cPBS, 0,1% BSA pendant au moins 45min à 4°C. Les cellules ont été lavées deux fois, suspendues dans 300µl de cPBS et analysées par FACS. Les anticorps utilisés sont indiqués dans le tableau 3 suivant :

**Tableau 3**

| | | Compagnies | Numéro catalogue | Dilution |
|---|---|---|---|---|
| Anticorps primaire | Polyclonal de lapin | Abcam | Ab3470 | 1/100 |
| | Monoclonal de souris | Abcam | Ab13248 | 1/100 |
| | Polyclonal de chèvre | R et D | AF3776 | 1/100 |
| Contrôle isotype | IgG de lapin | Sigma | I87765 | 0,016 |
| | IgG de souris | Sigma | 15381 | 0,020 |
| | IgG de chèvre | Sigma | I5256 | 0,020 |
| Anticorps secondaire | Ane anti-lapin PE | Immuno Research | 711-116-152 | 1/100 |
| | Chèvre anti souris PE | Santa-Cruz | SC-3738 | 1/100 |
| | Lapin anti chèvre PE | Santa-Cruz | SC-3755 | 1/100 |

### B Résultats

### B.1 Caractérisation générale des cellules

Les gels de transcrits d'ARNm issu des produits obtenus avec la RT-PCR ont montrés que les cellules exprimaient les gènes CXCL16, SDF-1, CX3CL1, 6CKine, CTACK (données non foumies).

Le gel issu de la PCR a montré que les cellules de la lignée MAgEC 11,5 exprimaient les gènes Kdr, Flt4 et CCR7 (colonnes 2, 4, 8, 10, 14 et 16, figure 16). Les cellules de la lignée MAgEC 10,5 exprimaient le gène Kdr (colonnes 1 et 2, figure 16).

Comme le montre la figure 8, qui représente des photographies de tests ELISA, toutes les cellules isolées selon le protocole décrit précédemment expriment les marqueurs vWf, ACE, et CD31 comme présenté dans l'exemple 1 précédent. Les cellules expriment également la VE-cadhérine qui est un marqueur de différentiation des cellules endothéliales. Cet exemple démontre donc clairement que les cellules obtenues sont des cellules précurseurs de cellules endothéliales félines.

De plus, comme démontré sur les figures 17 à 20, les lignées cellulaires MAgEC 10,5 et 11,5 n'expriment pas la chimiokine CCL27 (résultats non présentés) et produisent la chimiokine CXCL12 (figure 18). La lignée cellulaire MAgEC 10,5 ne produit pas de VEGF, CCL17, CCL27 et CXCL1 (figures 17, 19 et 20). L'expression de CCL17 est constante dans les cellules de la lignée MAgEC 11,5 quelque soit les conditions (hypoxie ou normoxie) tandis que la production de CXCL1 est diminuée lorsque les cellules sont en condition hypoxiques.

### B.2 Profils d'expression des lectines

Les figures 9 à 12 représentant l'intensité de fluorescence en fonction des cellules démontrent clairement que l'expression des molécules de type galectine et collectine change durant le stade de développement. Le niveau d'expression augmente à partir des cellules 10,5 dcp jusqu'à un pic pour les clones 6-1 (11,5 dcp) et une diminution dans les cellules des clones 4-3 (11,5 dcp). Tel que le montre les résultats obtenus, le niveau d'expression des lectines liant le rhamnose ne change pas entre les cellules indifférenciées des clones 6-1 et les cellules différenciées des clones 4-3. Ces résultats démontrent donc clairement que les récepteurs spécifiques du mannose-6P sont distribués ubiquitairement parmi les cellules de l'invention

### B. 3. Immunomarquage

L'immunomarquage des cellules de l'invention a révélé la présence des marqueurs AGM PODXL et EphB4 dérivés des cellules, tel que représenté dans la figure 12. Par ailleurs, le cluster de différentiation CD34 a été exprimé de manière croissante à partir des cellules 10,5 dpc aux cellules 11,5 dpc.

### Exemple 3 : criblage de molécules permettant l'identification de molécules susceptibles d'induire une différentiation et/ou une spécialisation d'une cellule isolée précurseur des cellules endothéliales

Les cellules précurseurs de cellules endothéliales humaines et murines sont mises en culture dans des plaques 24 puits (Falcon, Grenoble, France). Le milieu de culture est l'OptiMEM (marque de commerce) commercialisé par la société Invitrogen supplémenté avec 2 % de Sérum Bovin Foetal (FCS) commercialisé par la société Hyclon, Longan, Utah, USA.

Les cellules sont ensuite cultivées pendant 24 heures puis la molécule à cribler est introduite dans le milieu de culture.

Les cellules sont ensuite cultivées pendant une durée allant de 24 à 48 heures à une température de 37 °C.

Après 2 jours de culture, les cellules sont observées quant à leur viabilité, croissance, différentiation.

Des photos des cellules en croissance peuvent être prises avec le microscope Axiovert S (marque de commerce) commercialisé par la société Zeiss Jena, Germany équipé avec une caméra digitale Fine Pix 5602 (marque de commerce) commercialisé par la société Fuji Film. Les observations montrent que les cellules ont soit subies une toxicité, soit une activation, soit une différentiation, etc.

L'observation des cellules en croissance permet de mettre en évidence l'effet de la molécule ciblée.

Une caractérisation des cellules est également effectuée avec des anticorps spécifiques de marqueurs de différentiation, tel que cela est présenté dans l'exemple 1 précité.

Cette caractérisation démontre clairement que les cellules expriment des marqueurs de cellules endothéliales différenciées, ou bien font de l'angiogenèse, ou bien croissent plus vite, ou bien évoluent vers d'autres lignées telles que les adipocytes, muscles lisses, os, etc., indiquant que la molécule criblée induit une stimulation, apoptose ou différentiation.

### Exemple 4 : adressage de cellules précurseurs au niveau de site pathologiques

Les cellules précurseurs utilisées dans cet exemple sont les cellules isolées des exemples 1 et 2 précités.

Afin de confirmer l'adressage des cellules précurseurs de cellules endothéliales au niveau de sites pathologiques, dans le cas présent au niveau de tumeurs, des expériences d'imagerie *in vivo* sont conduites.

Des souris tumorales sont obtenues par injection de 10⁵ cellules de mélanome B16-F10 au niveau sous cutané, au-dessus de la patte, afin de former des tumeurs. Les souris utilisées sont de la race C57 BI6. Les souris obtenues constituent des modèles tumoraux.

La vérification de la présence des tumeurs est effectuée par mesure visuelle et matérielle de la taille de la tumeur.

Les cellules précurseurs de cellules endothéliales sont marquées avec le colorant DiD Vybrant (marque enregistrée) commercialisé par la société Molecular Probes USA

Après coloration, les cellules marquées sont injectées dans les souris porteuses de tumeurs.
L'acquisition des images est faite avec un appareil de mesure de la fluorescence dans le proche Infra Rouge, au Centre d'Imagerie du Petit Animal (CIPA) CNRS, Orléans, France.

Les images obtenues montrent clairement un adressage des cellules au niveau des tumeurs. Par ailleurs, les résultats obtenus montrent que les cellules ont été incorporées dans les tumeurs.

Une expérience similaire est effectuée chez des souris ayant subit un infarctus du myocarde.

Les souris utilisées sont identiques aux souris précitées et les cellules précurseurs de cellules endothéliales sont marquées selon le procédé décrit précédemment.

L'infarctus est provoqué par lésion d'une coronaire chez la souris. Après l'infarctus, les cellules précurseurs de cellules endothéliales marquées sont injectées dans les souris ayant subies un infarctus. La révélation des cellules est faite telle qu'indiquée précédemment.

Les images obtenues montrent clairement un adressage des cellules au niveau des tumeurs et également au niveau des tissus endommagés par l'infarctus.

Par ailleurs, les résultats obtenus montrent que les cellules sont incorporées dans les tumeurs et permet la restauration des tissus endommagés et la cicatrisation.

Tel que le démontre cet exemple, les cellules isolées précurseurs de cellules endothéliales selon l'invention, ciblent les tumeurs ainsi que les tissus endommagés. Aussi, ces cellules peuvent être utilisées, par exemple pour le traitement de lésions tissulaires, pour l'adressage de molécules thérapeutiques, par exemple au niveau de tumeurs et pour le traitement de cellules génétiquement déficientes.

### Exemple 5 : Ciblage de sites tumoraux et de vaisseaux en formation par des cellules précurseurs de cellules endothéliales

### 1. Matériel et Méthodes

### 1.1 Marquage par des nano-particules anioniques magnétiques (AMNP)

Les cellules pour l'imagerie par résonance magnétique ont été marquées par des AMNP appartenant au SPIO (Oxide de fer super paramagnétique) et au groupe des agents de contraste de résonance magnétique. Les cellules à 70% de confluence ont été rincées deux fois avec 8 ml de milieu de base OptiMEM (marque déposée) avec 5 mM de citrate de sodium. Ensuite, 5ml de milieu empty OptiMEM (marque déposée) avec 5 mM de citrate de sodium et 2mM de fluide ferrique (AMNP) ont été ajoutés au contenant de culture. Les cellules ont été incubées à 37°C, 5% de CO₂ pendant 15 à 20 minutes. Après incubation, les cellules ont été rincées deux fois avec du milieu de base OptiMEM (marque déposée) comprenant 5 mM de citrate de sodium. Le milieu avec le citrate a été retiré et 8ml de milieu de base OptiMEM (marque déposée) a été ajouté. Les cellules ont été incubées 2 heures dans un incubateur humidifié à 37°C, 5% de CO₂, puis le milieu a été changé pour un milieu complet OptiMEM (marque déposée). Les cellules ont été utilisées pour injections intraveineuses 24 heures après marquage.

### 1.2. Marquage PKH67

Les cellules pour marquage PKH67 ont été détachées avec 0,05% de trypsine EDTA (commercialisé par la société Gibco), bloquées avec le milieu complet OptiMEM (marque déposée). Après détachement, les cellules ont été lavées deux fois avec du milieu de base OptiMEM (marque déposée). Après le second lavage, les cellules ont été resuspendues dans 500µl de Diluent C commercialisé par la société New England BIOLABS, puis ajoutées à 500µl de Diluent C avec 4µl de PKH67 commercialisé par la société Invitrogen, et incubées à 37°C pendant 3 minutes. Après incubation, le marquage a été arrêté avec 0,5% de BSA dans du cPBS (phosphate buffered saline, complété par 1 mM CaCl2 et 0,5 MM MgCl2). Les cellules ont été lavées deux fois avec du tampon salin, comptées avec une chambre de Malassez et resuspendues à une concentration de 10x10⁶ cellules par ml.

### 1.3. Imagerie par Résonance magnétique

10 jours avant l'imagerie, 5x10⁵ cellules B16(F10) ont été injectées en sous-cutané dans des souris. Des cellules B16(F10) de mélanome ont été détachées avec 0,05% de trypsine EDTA commercialisé par la société Gibco, puis rincées deux fois avec 0,9% de NaCl (poids/volume) et comptées avec une chambre Malassez. Les cellules ont été resuspendues dans 0,9% de NaCl (poids/volume) à une concentration de 10x10⁶ cellules par millilitre et mélangées 1:1 (volume : volume) avec du Matrigel (marque déposée) commercialisé par la société BD biosciences, juste avant l'injection. Avant l'injection, les souris ont été anesthésiées avec 2-3 % d'isoflurane. Les membres des souris ont été rasés avec un rasoir électrique et épilés avec une crème d'épilation (crème épilation Dermotolérance, Vichy). 100µl de Matrigel (marque déposée) ont été injectés avec 5x10⁵ de cellules B16(F10) par membre.

Les cellules MAgEC 11.5 marquées avec de l'AMNP et du PKH67 ou de l'AMNP-Rhodamine ont été resuspendues dans 0.9% de NaCl (poids/volume) à une concentration de 10x10⁶ cellules par ml. 100µl de cellules suspendues ont été injectés en intraveineuse soit dans le sinus rétro-orbital soit dans la veine de la queue des souris présentant des tumeurs B16(F10) sous-cutanées. Une injection dans une souris de 1x10⁶ cellules MAgEC 11.5 fixées par 4% de paraformaldéhyde (Sigma) (poids volume) dans le PBS après incubation de 30 minutes à 37°C, et une injection de 100µl de 0,9% de NaCl (poids/volume) a été effectuée dans une souris pour le témoin. Les images des souris ont été prises avant et 24 heures après injections intraveineuses avec un spectroscope Brüker BioSpin 9,4T.

### 1.4. Isolement des cellules tumorales de la rate, des ganglions lymphatiques, des poumons

Les souris ont été sacrifiées par translocation cervicale. Les poumons, rate, ganglions lymphatiques mésentériques ont été isolés, dilacérés avec du PBS et incubés avec 5 mg/ml de collagénase de type I commercialisé par la société Invitrogen pendant 30 minutes à 37°C. Après incubation, les organes ont été lavés avec du PBS et dissociés sur des filtres et grilles et nylon. Après la dissociation, les érythrocytes ont été lysés trois fois avec de l'eau distillée. Brièvement, les cellules ont été centrifugées pendant 5 minutes à 1500 tours par minutes et resuspendues dans une goutte de PBS. 1 ml d'eau distillée a été ajouté à la suspension cellulaire. Après 10 secondes, 4 ml de PBS ont été ajoutés à chaque tube. Le culot de cellules a été resuspendu dans du PBS.

### 1.5. Imagerie in-vivo par fluorescence proche de l'infrarouge

10 jours avant l'imagerie 5x10⁵ de B16(F10)-*luc-eGFP,* cellules obtenues en collaboration avec le laboratoire de biotechnologies médicales de 'la faculté de biotechnologies de l'Université Jagellon de Cracovie, ont été injectés en sous-cutané. Les cellules de mélanomes ont été détachées avec 0,05% de trypsine EDTA commercialisée par la société Gibco, puis rincées deux fois avec 0,9% de NaCl (poids/volume) et comptées avec une chambre Malassez. Les cellules ont été resuspendues dans 0,9% de NaCl (poids/volume) à une concentration de 10x10⁶ cellules par millilitre et mélangées 1:1 (volume: volume) avec du Matrigel (marque déposée), juste avant l'injection. Avant l'injection, la souris a été anesthésiée avec de l'isoflurane. Les membres des souris ont été rasés avec un rasoir électrique et 100µl de Matrigel comprenant 5x10⁶ cellules B16(F10)-luc-eGFP ont été injectés en sous-cutané. 100µl de Matrigel ont été injectés en sous-cutané dans deux souris pour le témoin. 24 heures avant la première image, une injection soit dans un sinus retro-orbital, soit dans la veine de la queue de souris a été réalisée avec 2x10⁶ cellules:
a) MAgEC 11,5 vivantes et marquées avec du DiD Vybrant (marque déposée)
b) MAgEC 11,5 fixées et marquées avec du DiD Vybrant (marque déposée).

Une souris avec des cellules B16(F10)-luc-eGFP en sous-cutanés n'a pas été traitée. Avant l'imagerie par bioluminescence, 150 mg/kg de luciférine de luciole ont été injectés par voie intrapéritonéale. Les images des souris proches de l'infrarouge ont été obtenues avec un IVIS lumina system commercialisé par la société Caliper chaque jour pendant une période d'une semaine en commençant 24 heures avec l'injection intraveineuse. L'imagerie bioluminescense a été réalisée selon le protocole Xenogen IVIS 50 bioluminescent : marquage par luciférase d'une tumeur dans des souris.

### 2. Résultats :

### 2.1 Efficacité du marquage des cellules MAgEC

La figure 21 représente les résultats du marquage des cellules avec les nanoparticules AMNP couplées avec la Rhodamine et montre une localisation cytoplasmique de l'agent de contraste (Figures 21, A et C) représentée par les flèches blanches. La fluorescence était faible mais suffisante (figures 21 B et C). En outre, la localisation des nanoparticules était irrégulière et située en particulier autour du noyau.

L'efficacité du marquage des cellules MAgEC 11,5 avec les agents de contrastes magnétiques; en particulier avec les particules AMNP couplées avec la Rhodamine 123 (MAgEC 11,5 - AMNP -Rho) a été mesurée par cytométrie en flux. Les résultats obtenus ont montré un marquage total des MAgEC, 90,4%, avec l'agent de contraste.

Le tableau 4 ci-dessous reprend les différents résultats obtenus exprimés en pourcentage.

**Tableau 4**

| Cellules | Rho- (%) | Rho+ (%) |
|---|---|---|
| MAgEC 11,5 | 99,3 | 0,33 |
| MAgEC 11,5 - AMNP -Rho | 5,72 | 90,4 |
| MAgEC 11,5 - AMNP -Rho HEPES | 4,56 | 93,7 |

| | | |
|---|---|---|
| Rho- : pourcentage de cellules non marquées Rho+ : pourcentage de cellules marquées | | |

En outre, l'addition d'HEPES, qui stabilise le pH durant l'incubation des cellules avec les AMNP (MAgEC 11,5 - AMNP -Rho HEPES), n'a pas changé l'efficacité du marquage. Le marquage a en outre été confirmé par magnétophorèse cellulaire (résultats non fournis).

### 2.2 IRM et cytométrie en flux, démonstration de la migration des MAgEC à travers un site cancéreux 24 heures après injection intraveineuse

Dans cette expérience, des images de vaisseaux sanguins ont été obtenues par imagerie par résonnance magnétique tel que décrit précédemment (Figure 22). Les tumeurs sous-cutanées B16(F10) étaient, après 7 jours post inoculation, vascularisées et permettaient ainsi un accès aux cellules MAgEC 11.5 injectées. Toutefois, l'organisation du réseau vasculaire dans les tumeurs n'était pas parfaite (figure 22 A). En effet, les images des vaisseaux obtenues pour un muscle sain (figure 22 A) étaient différentes de celles obtenues pour une tumeur sous-cutanée (figure 22 B). En outre, le bruit de fond sur la figure 22 B indique une perméabilité vasculaire ce qui est un phénomène normal dans le contexte tumoral .

24 heures après injection, dans des souris présentant des tumeurs vasculaires B16(F10), de 1,0x10⁵ cellules MAgEC 11.5 marquées avec AMNP, des images ont été de nouveau prises afin d'étudier la migration des cellules modèles (MAgEC) vers le site cancéreux. Les images obtenues (figure 23) ont montré une région hypodense dans les tumeurs des souris injectées et non injectées.

Une analyse des images a été réalisée par comparaison de la densitométrie du signal dans la tumeur et dans le muscle. Cette comparaison ne montrait pas de différence statistique entre la souris sans et avec injection de cellules MAgEC 11,5 marquées avec AMNP. Ainsi, les inventeurs ont démontré que la vasculature désorganisée et chaotique des tumeurs, en particulier lors de la croissance rapide des mélanomes sous-cutanées B16(F10), doit être remplie de déoxyhémoglobine qui fournit un signal similaire aux agents de contrastes d'oxyde de fer superparamagnétiques. Ceci modifie le résultat attendu et n'a pas permis d'observer de différence entre les deux conditions. Toutefois, comme représenté sur la figure 23, une localisation préférentielle des cellules MAgEC et la formation de vaisseaux par ces cellules a été observée. En outre, la formation des vaisseaux par les cellules MAgEC est similaire à celle observée lors d'une néoangiogenèse et présente une structure cohérente et linéaire.

Cette expérience démontre donc clairement que les cellules précurseurs de la présente invention sont recrutées au niveau des tumeurs et permettent la formation de néovaisseaux. Ainsi, ces cellules permettent un ciblage efficace de tumeurs et de l'angiogenèse tumorale.

Afin de vérifier la distribution des cellules injectées dans la rate, les poumons, les noeuds lymphoïdes et les tumeurs d'animaux sans injection (contrôle) et d'animaux avec injection de cellules MAgEC 11.5, les organes ont été extraits et dissociés comme décrit précédemment. L'observation de la présence de cellules MAgEC 11.5 marquées avec de la Rhodamine ou de l'AMNP et PKH67 tel que décrit précédemment, a été réalisée par cytométrie en flux des cellules des organes disséqués. Dans toutes les conditions, 100 000 évènements ont été analysés.

La figure 24 représente les résultats obtenus pour chaque organe. En particulier, un nombre significatif de cellules marquées avec le PK67 a été trouvé dans les tumeurs et dans la rate en comparaison avec le bruit de fond (fluorescence sans marqueur), à savoir dans la rate: 0,47%± 0,15% par rapport à 0,072±0,025%, p=0,0121 et dans la tumeur 0,42%± 0,010% par rapport à 0,005±0,0012%, p=0,0039 (figure 24).

Cette expérience démontre donc clairement que les cellules MAgEC 11.5 peuvent cibler des tumeurs et également différents organes.

### 2.3 Localisation des cellules MAgEC 11.5, 7 jours après injection

Le protocole de mise en oeuvre correspond au protocole décrit au point 1.5 précité.

Les signaux obtenus par des cellules marquées par DiD par imagerie proche de l'infrarouge sont absorbés par la mélanine. Aussi, lors de la réalisation de cet exemple, il n'a pas été possible de détecter *in-vivo* les cellules MAgEC 11.5 sous une peau pigmentée et dans des tumeurs *B16-luc-eGFP.* Aussi, une souris de chaque groupe a été sacrifiée et scannée après résection de la peau et des intestins afin de réduire le signal de fluorescense de la chlorophylle.

Dans onze souris dans lesquelles des cellules MAgEC 11,5 vivantes et marquées avec du DiD Vybrant (marque déposée) ont été injectées, les cellules ont été détectées dans le foie (11/11 souris), la rate (5/11 souris), les noeuds lymphoïdes (3/11 souris), les poumons (2/11 souris), le sternum et le tibia comme représenté sur les figures 25 IA, IB et IIA et IIB. Une fluorescence faible a été détectée dans le foie, la rate les poumons et le sternum des souris où les cellules MAgEC 11,5 fixées et marquées avec du DiD Vybrant (marque déposée) ont été injectées. Aucune fluorescence n'a été détectée dans les souris dans lesquelles aucune injection n'a été réalisée (Figures 25 IC et IIC).

Cet exemple démontre donc clairement que les cellules de l'invention peuvent être utilisées pour le ciblage d'organes particuliers et peuvent être utilisées, par exemple dans des procédés de réparation tissulaire, des procédés de libération de substances actives et ou de gènes dans différents organes.

### SEQUENCE LISTING

<110> Centre National de la Recherche Scientifique (CNRS)
   Ludwik HIRSZFELD Institute of Immunology and Experimental
   Therapy
   kieda, claudine
   Grillon, Catherine
   Lamerant, Nathalie
   Praprocka, Maria
   Dus, Danuta
   Krawczenko, Agnieszka
<120> Lignes de cellules souches humaines et murines: modle de prcurseur de cellules endothliales
<130> BIP133632PC00
<140> PCT/FR2011/0500xx
   <141> 2011-01-11
<150> FR 10/00111
   <151> 2010-01-12
<160> 30
<170> PatentIn version 3.5
<210> 1
   <211> 24
   <212> DNA
   <213> Artificial Sequence
<220>
   <223> amorce MDR-1s
<400> 1
   aagcttagta ccaaagaggc tctg 24
<210> 2
   <211> 24
   <212> DNA
   <213> Artificial Sequence
<220>
   <223> amorce MDR-1as
<400> 2
   ggctagaaac aatagtgaaa acaa 24
<210> 3
   <211> 20
   <212> DNA
   <213> Artificial Sequence
<220>
   <223> amorce MDR3-s
<400> 3
   agggcgactt tgaactgggc 20
<210> 4
   <211> 20
   <212> DNA
   <213> Artificial Sequence
<220>
   <223> amorce MDR3-as
<400> 4
   tttgcctgga tttagcagcg 20
<210> 5
   <211> 25
   <212> DNA
   <213> Artificial Sequence
<220>
   <223> amorce MRP1-s
<400> 5
   agtgacctct ggtccttaaa caagg 25
<210> 6
   <211> 25
   <212> DNA
   <213> Artificial Sequence
<220>
   <223> amorce MRP1-as
<400> 6
   gaggtagaga gcaaggatga cttgc 25
<210> 7
   <211> 19
   <212> DNA
   <213> Artificial Sequence
<220>
   <223> amorce BCRP-s
<400> 7
   ttaggattga agccaaagg 19
<210> 8
   <211> 21
   <212> DNA
   <213> Artificial Sequence
<220>
   <223> amorce BCRP-as
<400> 8
   taggcaattg tgaggaaaat a 21
<210> 9
   <211> 24
   <212> DNA
   <213> Artificial Sequence
<220>
   <223> amorce LRP-s
<400> 9
   gtcttcgggc ctgagctggt gtcg 24
<210> 10
   <211> 24
   <212> DNA
   <213> Artificial Sequence
<220>
   <223> amorce LRP-as
<400> 10
   cttggccgtc tcttgggggt cctt 24
<210> 11
   <211> 20
   <212> DNA
   <213> Artificial Sequence
<220>
   <223> amorce actin-s
<400> 11
   ccagagcaag agaggcatcc 20
<210> 12
   <211> 20
   <212> DNA
   <213> Artificial Sequence
<220>
   <223> amorce actin-as
<400> 12
   ctgtggtggt gaagctgaag 20
<210> 13
   <211> 20
   <212> DNA
   <213> Artificial Sequence
<220>
   <223> amroce sens CXCL16
<400> 13
   agacagcaag aagcaccagg 20
<210> 14
   <211> 20
   <212> DNA
   <213> Artificial Sequence
<220>
   <223> amorce antisens CXCL16
<400> 14
   cctctcccat gtcatcatcc 20
<210> 15
   <211> 20
   <212> DNA
   <213> Artificial Sequence
<220>
   <223> amorce sens SDF-1
<400> 15
   gttcttcgag agccacatcg 20
<210> 16
   <211> 20
   <212> DNA
   <213> Artificial Sequence
<220>
   <223> amroce SDF-1 antisens
<400> 16
   atggcggagt gtctttatgc 20
<210> 17
   <211> 20
   <212> DNA
   <213> Artificial Sequence
<220>
   <223> amorce sens CX3CL1
<400> 17
   tgcgacaaga tgacctcacg 20
<210> 18
   <211> 20
   <212> DNA
   <213> Artificial Sequence
<220>
   <223> amorce CX3CL1 antisens
<400> 18
   atcctgtgcc tcggaagttg 20
<210> 19
   <211> 22
   <212> DNA
   <213> Artificial Sequence
<220>
   <223> amorce sens 6Ckine
<400> 19
   ggactgctgc cttaagtaca gc 22
<210> 20
   <211> 21
   <212> DNA
   <213> Artificial Sequence
<220>
   <223> amroce 6Ckine antisens
<400> 20
   gctatcctct tgagggctgt g 21
<210> 21
   <211> 23
   <212> DNA
   <213> Artificial Sequence
<220>
   <223> amorce sens CTACK
<400> 21
   ggctgagtga gtgagcatga tgg 23
<210> 22
   <211> 20
   <212> DNA
   <213> Artificial Sequence
<220>
   <223> amorce CTACK antisens
<400> 22
   gggatgaaca cagacactgc 20
<210> 23
   <211> 20
   <212> DNA
   <213> Artificial Sequence
<220>
   <223> amorce sens Kdr
<400> 23
   agagtgtgtc cctgttgtgc 20
<210> 24
   <211> 20
   <212> DNA
   <213> Artificial Sequence
<220>
   <223> amorce antisens Kdr
<400> 24
   ggggtaggat ttccagatgc 20
<210> 25
   <211> 20
   <212> DNA
   <213> Artificial Sequence
<220>
   <223> amroce sens Flt4
<400> 25
   gagtgactcc ctggagatgc 20
<210> 26
   <211> 20
   <212> DNA
   <213> Artificial Sequence
<220>
   <223> amorce antisens Flt4
<400> 26
   tagcccgtct tgatgtctgc 20
<210> 27
   <211> 19
   <212> DNA
   <213> Artificial Sequence
<220>
   <223> amroce sens CCR7
<400> 27
   aaacccagga aaaacgtgc 19
<210> 28
   <211> 21
   <212> DNA
   <213> Artificial Sequence
<220>
   <223> amorce antisens CCR7
<400> 28
   rgtagacgcc aaagatccag g 21
<210> 29
   <211> 20
   <212> DNA
   <213> Artificial Sequence
<220>
   <223> amorce sens CXC3R1
<400> 29
   tcttcatcac cgtcatcagc 20
<210> 30
   <211> 20
   <212> DNA
   <213> Artificial Sequence
<220>
   <223> amorce antisens CXC3R1
<400> 30
   gatgcggaag tagcaaaagc 20

### Listes des références

1. Arbab AS, Pandit SD, Anderson SA, et al. Magnetic resonance imaging and confocal microscopy studies of magnetically labeled endothelial progenitor cells trafficking to sites of tumor angiogenesis. Stem Cells. 2006;24:671-678
2. de Jonge-Peeters SD, Kuipers F, de Vries EG, Vellenga E. « ABC transporter expression in hematopoietic stem cells and the role in AML drug resistance. » Crit Rev Oncol Hematol. 2007 Jun; 62(3):214-26. Epub 2007 Mar 23.
3. Slayton WB, Li XM, Butler J, Guthrie SM, Jorgensen ML, Wingard JR, Scott EW. « The role of the donor in the repair of the marrow vascular niche following hematopoietic stem cell transplant. » Stem Cells. 2007 Nov;25(11):2945-55
4. Dome B, Timar J, Ladanyi A, et al. Circulating endothelial cells, bone marrow-derived endothelial progenitor cells and proangiogenic hematopoietic cells in cancer: From biology to therapy. Crit Rev Oncol Hematol. 2009;69:108-1241
5. Yamahara K, Itoh H. Potential use of endothelial progenitor cells for regeneration of the vasculature. Ther Adv Cardiovasc Dis. 2009;3:17-27.
6. Suh W, Kim KL, Kim JM, et al. Transplantation of endothelial progenitor cellsaccelerates dermal wound healing with increased recruitment of monocytes/macrophages and neovascularization. Stem Cells. 2005;23:1571-1578.
7. Slayton WB, Li XM, Butler J, et al. The role of the donor in the repair of the marrowvascular niche following hematopoietic stem cell transplant. Stem Cells.2007;25:2945-2955
8. Shi Q, Rafii S, Wu MH, et al. Evidence for circulating bone marrow-derived endothelial cells. Blood. 1998;92:362-367
9. Lin Y, Weisdorf DJ, Solovey A, et al. Origins of circulating endothelial cells and endothelial outgrowth from blood. J Clin Invest. 2000;105:71-77.
10. Peichev M, Naiyer AJ, Pereira D, et al. Expression of VEGFR-2 and AC133 by circulating human CD34(+) cells identifies a population of functional endothelial precursors. Blood. 2000;95:952-958.
11. Case J, Mead LE, Bessler WK, Prater D, White HA, Saadatzadeh MR, Bhavsar JR, Yoder MC, Haneline LS, Ingram DA « Human CD34+AC133+VEGFR-2+ cells are not endothelial progenitor cells but distinct, primitive hematopoietic progenitors. » Exp Hematol. 2007 Oct;35(10):1479-80
12. Asahara T, Murohara T, Sullivan A, Silver M, van der Zee R, Li T, Witzenbichler B, Schatteman G, Isner JM. « Isolation of putative progenitor endothelial cells for angiogenesis » Science. 1997 Feb 14;275(5302):964-7
13. Szyda A, Paprocka M, Krawczenko A, et al. Optimization of a retroviral vector for transduction of human CD34 positive cells. Acta Biochim Pol. 2006;53:815-823.
14. Kieda C, Paprocka M, Krawczenko A, Za 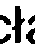 ecki P, Dupuis P, Monsigny M, Radzikowski C, Dus D., New human microvascular endothelial cell lines with specific adhesion molecules phenotypes. Endothelium. 2002;9(4):247-61.

## Revendications

1. Cellule isolée précurseur des cellules endothéliales choisie parmi les cellules isolées humaines précurseur de cellules endothéliales déposées à la Collection Nationale de Culture de Microorganismes (CNCM), sous les numéros CNCM n° I-4220, n° I-4221 ou les cellules isolées murines déposées à la Collection Nationale de Culture de Microorganismes (CNCM), sous les numéros CNCM n° I-4222 n° I-4223.

2. Cellule isolée selon la revendication 1, dans laquelle ladite cellule forme une lignée cellulaire établie.

3. Procédé de criblage de molécules susceptibles d'induire une différentiation et/ou une spécialisation d'une cellule isolée selon la revendications 1 ou 2 comprenant les étapes suivantes :
- introduction de la molécule à cribler dans un milieu adapté à la culture de ladite cellule,
- introduction d'au moins une cellule isolée selon la revendication 1 ou 2 dans ledit milieu,
- culture de ladite cellule dans ledit milieu pendant un temps suffisant pour permettre sa différentiation et/ou sa spécialisation,
- prélèvement d'au moins une cellule issue de ladite culture,
- observation de la prolifération ou non différentiation et/ou spécialisation de ladite cellule par observation phénotypique de la cellule et/ou par révélation de marqueurs de différentiation.

4. Procédé selon la revendication 3, dans lequel la révélation de marqueurs de différentiation est effectuée au moyen d'anticorps spécifiques desdits marqueurs.

5. Procédé selon la revendication 3 ou 4, dans lequel lesdites molécules induisent une différentiation et/ou spécialisation conduisant à l'angiogenèse.

6. Procédé selon l'une quelconque des revendications 3 à 5, dans lequel l'étape d'identification du phénotype et/ou des marqueurs permet de connaître l'effet de la molécule criblée sur la cellule isolée cultivée.

7. Kit de mise en oeuvre du procédé selon la revendication 3 comprenant :
- au moins une cellule isolée selon la revendication 1 ou 2, et
- un moyen de détection de la différentiation et/ou spécialisation cellulaire.

8. Culture cellulaire comprenant une cellule isolée selon la revendications 1 ou 2.

9. Cellule isolée selon la revendications 1 ou 2 pour l'utilisation comme médicament.

10. Utilisation d'une cellule isolée selon la revendications 1 ou 2 pour la fabrication d'un médicament.

11. Utilisation d'une cellule isolée selon la revendication 10, dans laquelle le médicament est destiné au traitement des cellules génétiquement déficientes, des lésions tissulaires, des dommages endothéliaux, des dommages dus à l'ischémie, l'infarctus, les tumeurs, le diabète.

## Patentansprüche

1. Isolierte Vorläuferzelle von Endothelzellen, die aus den isolierten humanen Vorläuferzellen von Endothelzellen, die bei der Collection Nationale de Culture de Microorganismes (CNCM) unter den CNCM-Nummern Nr. I-4220, Nr. I-4221 hinterlegt sind, oder den isolierten murinen Zellen, die bei der Collection Nationale de Culture de Microorganismes (CNCM) unter den CNCM-Nummern Nr. I-4222, Nr. I-4223 hinterlegt sind, ausgewählt ist.

2. Isolierte Zelle nach Anspruch 1, wobei die besagte Zelle eine etablierte Zelllinie bildet.

3. Verfahren zum Screenen von Molekülen, die dazu imstande sind, eine Differenzierung und/oder eine Spezialisierung einer isolierten Zelle nach den Ansprüchen 1 oder 2 zu induzieren, wobei das Verfahren die folgenden Schritte umfasst:
- Einbringen des zu screenenden Moleküls in ein Medium, das zur Kultivierung der besagten Zelle geeignet ist,
- Einbringen mindestens einer isolierten Zelle nach Anspruch 1 oder 2 in das besagte Medium,
- Kultivieren der besagten Zelle in dem besagten Medium für einen Zeitraum, der dazu ausreicht, ihre Differenzierung und/oder ihre Spezialisierung zu ermöglichen,
- Entnehmen mindestens einer Zelle, die aus der besagten Kultur hervorgeht,
- Beobachten der Proliferation oder Nichtdifferenzierung und/oder Spezialisierung der besagten Zelle durch phänotypische Beobachtung der Zelle und/oder durch Offenbarung von Differenzierungsmarkern.

4. Verfahren nach Anspruch 3, wobei die Offenbarung von Differenzierungsmarkern mithilfe spezifischer Antikörper der besagten Marker vorgenommen wird.

5. Verfahren nach Anspruch 3 oder 4, wobei die besagten Moleküle eine Differenzierung und/oder Spezialisierung induzieren, die zu einer Angiogenese führt.

6. Verfahren nach einem der Ansprüche 3 bis 5, wobei der Schritt zur Identifizierung des Phänotyps und/oder von Markern ermöglicht, die Wirkung des gescreenten Moleküls auf die kultivierte isolierte Zelle zu erfahren.

7. Kit zur Durchführung des Verfahrens nach Anspruch 3, wobei das Kit Folgendes umfasst:
- mindestens eine isolierte Zelle nach Anspruch 1 oder 2 und
- ein Mittel zum Nachweisen der Zelldifferenzierung und/oder -Spezialisierung.

8. Zellkultur, die eine isolierte Zelle nach den Ansprüchen 1 oder 2 umfasst.

9. Isolierte Zelle nach den Ansprüchen 1 oder 2 zur Verwendung als Arzneimittel.

10. Verwendung einer isolierten Zelle nach den Ansprüchen 1 oder 2 zur Herstellung eines Arzneimittels.

11. Verwendung einer isolierten Zelle nach Anspruch 10, wobei das Arzneimittel zur Behandlung von genetisch defekten Zellen, Gewebeläsionen, Endothelschäden, Schäden aufgrund einer Ischämie, einem Herzinfarkt, von Tumoren, von Diabetes vorgesehen ist.

## Claims

1. An isolated endothelial cell precursor cell selected form the group comprising the isolated human endothelial cell precursor cells deposited at the Collection Nationale de Cultures de Microorganismes (CNCM), under CNCM numbers No. I-4220, No. I-4221 or the isolated murine endothelial cell precursor cells deposited at the Collection Nationale de Cultures de Microorganismes (CNCM), under CNCM numbers No. I-4222 No. I-4223.

2. The isolated cell according to claim 1, wherein said cell forms an established cell line.

3. A method for screening molecules that can induce differentiation and/or specialization of an isolated cell according to claim 1 or 2 comprising the following steps:
- introducing the molecule to be screened into a medium that is suitable for culture of said cell,
- introducing at least one isolated cell according to claim 1 or 2 in said medium,
- culturing said cell in said medium for a sufficient time to allow its differentiation and/or its specialization,
- taking at least one cell resulting from said culture,
- observation of the proliferation or non-differentiation and/or specialization of said cell by phenotypic observation of the cell and/or by detecting differentiation markers.

4. The method according to claim 3, wherein the detection of differentiation markers is carried out by means of specific antibodies of said markers.

5. The method according to claim 3 or 4, wherein said molecules induce differentiation and/or specialization leading to angiogenesis.

6. The method according to any one of claim 3 to 5, wherein the step of identification of the phenotype and/or of the markers shows the effect of the screened molecule on the cultured isolated cell.

7. A kit for carrying out the method according to claim 3 comprising:
- at least one isolated cell according to any one of claims 1 to 2, and
- means for detecting cellular differentiation and/or specialization.

8. A cell culture comprising an isolated cell according to any one of claims 1 to 4.

9. The isolated cell according to claim 1 or 2 for use as a medicinal product.

10. The use of an isolated cell according to claim 1 or 2 for manufacturing a medicinal product.

11. The use of an isolated cell according to claim 11, wherein the medicinal product is intended for treating genetically deficient cells, tissue lesions, endothelial damage, damage due to ischemia, infarction, tumors, diabetes.
